(19) Europäisches Patentamt
European Patent Office
Office européen des brevets

(11) Veröffentlichungsnummer: **0 227 717**
**B1**

(12) # EUROPÄISCHE PATENTSCHRIFT

(45) Veröffentlichungstag der Patentschrift: **25.04.90**

(51) Int. Cl.⁵: **C 07 D 239/34,**
C 07 D 239/26, C 09 K 19/34

(21) Anmeldenummer: **86903333.2**

(22) Anmeldetag: **16.05.86**

(86) Internationale Anmeldenummer:
**PCT/EP86/00293**

(87) Internationale Veröffentlichungsnummer:
**WO 86/07055 04.12.86 Gazette 86/26**

(54) **STICKSTOFFHALTIGE HETEROCYCLISCHE ESTER.**

(30) Priorität: **24.05.85 DE 3518734**

(43) Veröffentlichungstag der Anmeldung:
**08.07.87 Patentblatt 87/28**

(45) Bekanntmachung des Hinweises auf die Patenterteilung:
**25.04.90 Patentblatt 90/17**

(84) Benannte Vertragsstaaten:
**CH DE FR GB LI**

(56) Entgegenhaltungen:
**EP-A-0 025 119**
**WO-A-86/04060**
**JP-A-56 164 170**
**JP-A-56 164 171**

(73) Patentinhaber: **MERCK PATENT GESELLSCHAFT MIT BESCHRÄNKTER HAFTUNG**
**Frankfurter Strasse 250 Postfach 4119**
**D-6100 Darmstadt (DE)**

(72) Erfinder: **KRAUSE, Joachim, Dr.,**
**Samuel-Morse-Strasse 14**
**D-6110 Dieburg (DE)**
Erfinder: **WÄCHTLER, Andreas,Dr.,**
**Goethestrasse 34**
**D-6103 Griesheim (DE)**
Erfinder: **REIFFENRATH, Volker**
**Pfungstädter Strasse 31**
**D-6100 Darmstadt-Eberstadt (DE)**
Erfinder: **SCHEUBLE, Bernhard,Dr.,**
**Am Grenzweg 18**
**D-6146 Alsbach (DE)**
Erfinder: **HITTICH, Reinhard,Dr.,**
**Am Kirchberg 11**
**D-6101 Modautal 1 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft stickstoffhaltige heterocyclische Ester der Formel IV

$$R^1—A^1—Z^1—A^2—[Z^2—A^3]_m—R^2 \qquad IV$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können, einer der Reste $R^1$ und $R^2$ auch H, F, Cl, Br oder CN,

$A^1$ ein unsubstituiertes oder durch ein oder zwei F- und/oder Cl- und/oder Br-Atome und/oder $CH_3$- und/oder CN-Gruppen substituiertes Strukturelement ausgewählt aus der Gruppe der Formeln 1 bis 4:

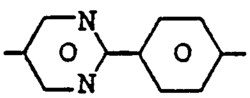 , 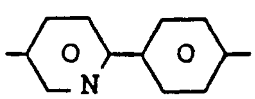 , 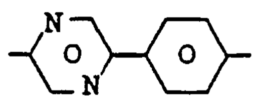 , 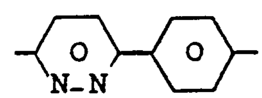

1 2 3 4

$A^2$ und $A^3$ jeweils trans-1,4-Cyclohexylen oder unsubstituiertes oder durch ein oder zwei F- und/oder Cl-Atome und/oder Br-Atome und/oder $CH_3$- und/oder CN-Gruppen substituiertes 1,4-Phenylen, worin auch eine oder mehrere CH-Gruppen durch N ersetzt sein können,

$Z^1$ —CO—O— oder —O—CO—,

$Z^2$ —CO—O—, —O—CO—, —$CH_2CH_2$—, —$CH_2O$—, —O—$CH_2$— oder eine Einfachbindung, und

m 0 oder 1

bedeuten, mit den Maßgaben, daß

(1) die Verbindungen mindestens eine lateral substituierte 1,4-Phenylen-Gruppe enthält und

(2) im Falle

$m = 0$ und $R^1$-$A^1$-$Z^1$ = Alkyl-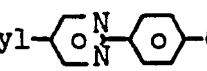-COO

$A^2$ lateral durch Fluor substituiertes 1,4-Phenylen oder in ortho-Stellung zu $R^2$ durch Cl, Br, $CH_3$ oder CN substituiertes 1,4-Phenylen bedeutet.

Chirale getiltete smektische flüssigkristalline Phasen mit ferroelektrischen Eigenschaften können hergestellt werden, indem man Basis-Mischungen mit einer oder mehreren getilteten smektischen Phasen mit einem geeigneten chiralen Dotierstoff versetzt (L. A. Beresnev et al., Mol. Cryst. Liq. Cryst. *89*, 327 (1982); H. R. Brand et al., J. Physique *44* (lett.) L-771 (1983). Solche Phasen können als Dielektrika für schnell schaltende Displays verwendet werden, die auf dem von Clark und Lagerwall beschriebenen Prinzip der SSFLC-Technologie (N. A. Clark und S. T. Lagerwall, Appl. Phys. Lett. *36*, 899 (1980); USP 4,367,924) auf der Basis der ferroelektrischen Eigenschaften der chiral getilteten Phasen beruhen. In dieser Phase sind die langgestreckten Moleküle in Schichten angeordnet, wobei die Moleküle einen Tiltwinkel zur Schichtennormalen aufweisen. Beim Fortschreiten von Schicht zu Schicht ändert sich die Tiltrichtung um einen kleinen Winkel bezüglich einer senkrecht zu den Schichten stehenden Achse, so daß eine Helixstruktur ausgebildet wird. In Displays, die auf dem Prinzip der SSFLC-Technologie beruhen, sind die smektischen Schichten senkrecht zu den Platten der Zelle angeordnet. Die helixartige Anordnung der Tiltrichtungen der Moleküle wird durch einen sehr geringen Abstand der Platten (ca. 1—2 µm) unterdrückt. Dadurch werden die Längsachsen der Moleküle gezwungen, sich in einer Ebene parallel zu den Platten der Zelle anzuordnen, wodurch zwei ausgezeichnete Tiltorientierungen entstehen. Durch Anlegen eines geeigneten elektrischen Wechselfeldes kann in der eine spontane Polarisation aufweisenden flüssigkristallinen Phase zwischen diesen beiden Zuständen hin- und hergeschaltet werden. Dieser Schaltvorgang ist wesentlich schneller als bei herkömmlichen verdrillten Zellen (TN-LCD's), die auf nematischen Flüssigkristallen basieren.

Ein großer Nachteil für viele Anwendungen der derzeit verfügbaren Materialien mit chiral getilteten smektischen Phasen (wie z.B. Sc*) ist deren geringe chemische, thermische und Photo-Stabilität. Eine weitere nachteilige Eigenschaft von Displays basierend auf derzeit verfügbaren chiral getilteten smektischen Mischungen ist, daß die Spontanpolarisation zu kleine Werte aufweist, so daß das Schaltzeitverhalten der Displays ungünstig beeinflußt wird und/oder der Pitch und der Tilt der Phasen nicht der Anforderungen der Display-Technologie entspricht. Darüberhinaus ist meist der Temperaturbereich der ferroelektrischen Phasen zu klein und liegt überwiegend bei zu hohen Temperaturen.

Es wurde nun gefunden, daß die Verwendung von Verbindungen der Formel IV als Komponenten chiral getilteter smektischer Mischungen die erwähnten Nachteile wesentlich vermindern kann. Die Verbindungen der Formel IV sind somit als Konponenten chiral getilteter smektischer flüssigkristalliner Phasen vorzüglich geeignet. Insbesondere sind mit ihrer Hilfe chemisch besonders stabile chiral getiltete

smektische flüssigkristalline Phasen mit günstigen ferroelektrischen Phasenbereichen, insbesondere mit breiten Sc* Phasenbereichen, hervorragender Unterkühlbarkeit bis zu Temperaturen weit unter 0°C, ohne daß Kristallisation auftritt (selbst erfindungsgemäße Phasen mit einem Schmelzpunkt oberhalb 0°C sind im allgemeinen bis weit unter 0°C unterkühlbar), günstiger Pitchhöhe und für derartige Phasen hohen Werten für die spontane Polarisation herstellbar. P ist die spontane Polarisation in $nC/cm^2$.

Ähnliche Verbindungen sind z.B. aus der DE—OS 35 06 446 bekannt. Die dort angegebenen Verbindungen sind jedoch im Gegensatz zu den vorliegenden keine Esterverbindungen. Aus JP—A—56—164170, JP—A—56—164171, WO—A—86/04060 und EP—A—25119 sind ebenfalls ähnliche Verbindungen bekannt. Die erfindungsgemäßen Verbindungen der Formel IV unterscheiden sich von den vorbekannten Verbindungen durch zusätzliche flüssigkristalline Eigenschaften, d.h., durch die Verwendbarkeit für chirale getiltete smektische flüssigkristalline Phasen mit ferroelektrischen Eigenschaften. Die Verwendung der Verbindungen der Formel IV als Komponenten derartiger Phasen, solche Phasen selbst sowie ferroelektrische Anzeigeelemente enthaltend derartige Phasen sind Gegenstand der Internationalen Patentanmeldung WO 86/07085.

Die Verbindungen der Formel IV können wie ähnliche Verbindungen als chirale Dotierstoffe für flüssigkristalline Phasen, insbesondere jedoch als Komponenten ferroelektrischer flüssigkristalliner Phasen verwendet werden. Diese Phasen eignen sich für Displays, die auf dem Prinzip der verdrillten Zelle (TN-Displays), dem Guest-Host-Effekt, dem Effekt der Deformation aufgerichteter Phasen oder dem Effekt der dynamischen Streuung beruhen, insbesondere jdedoch für ferroelektrische Displays beispielsweise nach N.A. Clark und S. T. Lagerwall, Applied Phys. Lett. *36*, 899 (1980).

Die Verbindungen der Formel IV besitzen einen breiten Anwendungsbereich. In Abhängigkeit von der Auswahl der Substituenten können diese Verbindungen als Basismaterialien dienen, aus denen flüssigkristalline smektische Phasen zum überwiegenden Teil zusammengesetzt sind; es können aber auch Verbindungen der Formel IV flüssigkristallinen Basismaterialien aus anderen Verbindungsklassen zugesetzt werden, um beispielsweise die dielektrische und/oder optische Anisotropie und/oder die Viskosität und/oder die spontane Polarisation und/oder die Phasenbereiche und/oder den Tiltwinkel und/ oder den Pitch eines solchen Dielektrikums zu variieren.

Die Verbindungen der Formel IV besitzen eine hohe chemische Stabilität. Sie sind farblos und gut mischbar mit allen gebräuchlichen Flüssigkristallen. Ihr Einsatz in flüssigkristallinen Phasen führt zu breiteren Mesophasenbereichen und verbesserten Werten für die Spontanpolarisation in chiral geilteten smektischen Phasen. Die erfindungsgemäßen Phasen eignen sich somit sehr gut für flüssigkristalline Phasen für Displays, die auf dem Prinzip der SSFLC-Technologie beruhen. Ferner eignen sie sich jedoch auch für andere elektrooptische Anzeigevorrichtungen, wie beispielsweise TN-Zellen oder Guest-Host-Zellen. Hier dienen sie neben der Erweiterung des Mesophasenbereichs insbesondere zur Vermeidung von reverse twist und zur Verbesserung der eleastischen Konstanten.

Gegenstand der Erfindung sind somit die Verbindungen der Formel IV.

Der Einfachheit halber bedeuten im folgenden Cy eine 1,4-Cyclohexylengruppe und Phe eine 1,4-Phenylengruppe, die gegebenenfalls auch durch ein oder zwei F- und/oder Cl-Atome und/oder $CH_3$- und/ oder CN-Gruppen substituiert sein kann (= Phe(F), Phe(Cl), Phe($CH_3$), Phe(CN)).

In den Verbindungen der vor- und nachstehenden Formeln bedeuten eine substituierte Alkylgruppe bzw. substituiertes Ethylen eine durch Halogen, vorzugsweise Fluor oder Chlor, oder CN ein oder mehrfach an verschiedenen C-Atomen substituierte Alkylgruppe bzw. $—CH_2CH_2—$ (Ethylen) -Gruppe. Vorzugsweise ist die Alkylgrupe nur einfach durch Halogen oder CN substituiert. Das mit Halogen oder CN verknüpfte C-Atom ist vorzugsweise ein asymmetrisches Kohlenstoffatom.

Einer der Reste $R^1$ und $R^2$ bedeutet vorzugsweise Alkyl, —O-Alkyl oder Oxaalkyl, —COO-Alkyl, —OCO-Alkyl, —CO-Alkyl oder Alkenyl.

Alkenylgruppen in den Verbindungen der Formel IV sind vorzugsweise geradkettige trans-Alkenylgruppen der Formel

$$CH_3-(CH_2)_{n1}-CH{=}HC-(CH_2)_{n2}-$$

worin

n2 0 bis 10, vorzugsweise 2 bis 10, und

n1 0 bis 5, vorzugsweise 0, bedeutet.

Die Alkylreste in den Gruppen $R^1$ und/oder $R^2$ können geradkettig oder verzweigt sein. Vorzugsweise sind sie geradkettig, haben 5, 6, 7, 8, 9, 10, 11 oder 12 C-Atome und bedeuten demnach bevorzugt Pentyl, Hexyl, Heptyl, Octyl, Nonyl, Decyl, Undecyl oder Dodecyl, ferner Methyl, Ethyl, Propyl, Butyl.

Falls $R^1$ und/oder $R^2$ Alkylreste bedeuten, in denen eine ("Alkoxy" bzw. "Oxaalkyl") oder zwei ("Alkoxyalkoxy" bzw "Dioxaalkyl") $CH_2$-Gruppen durch O-Atome ersetz sind, so können sie geradkettig oder verzweigt sein. Vorzugsweise sind sie geradkettig, haben 5, 6, 7, 8, 9, 10, 11 oder 12 C-Atome und bedeuten demnach bevorzugt Pentoxy, Hexoxy, Heptoxy, Octoxy, Nonoxy, Decoxy, Undecoxy, Dodecoxy, 2-, 3- oder 4-Oxapentyl, 2-, 3-, 4- oder 5-Oxahexyl. 2-, 3-, 4-, 5- oder 6-Oxaheptyl, 2-, 3-, 4-, 5-, 6- oder 7-Oxaoctyl, 2-, 3-,

4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl, 1,4-Dioxaoctyl, 1,4,7-Trioxaoctyl, 1,4-Dioxanonyl, 1,4-Dioxadecyl.

Verbindungen der Formel IV sowie der vor- und nachstehenden Teilformeln mit verzweigten Flügelgruppen $R^1$ bzw. $R^2$ können gelegentlich wegen einer besseren Löslichkeit in den üblichen flüssigkristallinen Basismaterialien von Bedeutung sein, insbesondere aber als chirale Dotierstoffe für chiral getiltete smektische Phasen, wenn sie optisch aktiv sind. Solche Verbindungen eignen sich jedoch auch als Komponenten nematischer flüssigkristalliner Phasen, insbesondere zur Vermeidung von reverse twist. Verzweigte Gruppen dieser Art enthalten in der Regel nicht mehr als eine Kettenverzweigung. Bevorzugte verzweigte Reste sind Isopropyl, 2-Butyl (= 1-Methylpropyl), Isobutyl (= 2-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, 2-Octyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy, 2-Oxa-3-methylbutyl, 3-Oxa-4-methylpentyl, 2-Octyloxy.

Vorzugsweise ist das asymmetrische Kohlenstoffatom mit zwei unterschiedlich substituierten C-Atomen, einem H-Atom und einem Substituenten ausgewählt aus der Gruppe Halogen (insbesondere F, Cl oder Br), Alkyl oder Alkoxy mit jeweils 1 bis 5 C-Atomen und CN verknüpft. Der optisch aktive organische Rest hat vorzugsweise die Formel,

```
         *
—X—Q—CH—R,
        |
        Y
```

worin

X —CO—O—, —O—CO—, —O—CO—O—, —CO—, —O—, —S—, —CH=CH— —CH=CH—COO— oder eine Einfachbindung,

Q Alkylen mit 1 bis 5 C-Atomen, worin auch eine nicht mit X verknüpfte $CH_2$-Gruppe durch —O—, —CO—, —O—CO—, —CO—O— oder —CH=CH— ersetzt sein kann, oder eine Einfachbindung,

Y CN, Halogen, Methyl oder Methoxy, und

R eine von Y vrschiedene Alkylgruppe mit 1 bis 18 C-Atomen, worin auch eine oder zwei nicht benachbarte $CH_2$-Gruppen durch —O—, —CO—, —O—CO—, —CO—O— und/oder —CH=CH— ersetzt sein können,

bedeutet.

X ist vorzugsweise —CO—O—, —O—CO—, —O—, —CH=CH—COO— (trans) oder eine Einfachbindung. Besonders bevorzugt sind —O—, —CO—O— und —O—CO—.

Q ist vorzugsweise Alkylen mit 1 bis 5 C-Atomen oder eine Einfachbindung, insbesondere bevorzugt —$CH_2$—, —$CH_2CH_2$— oder eine Einfachbindung.

Y ist vorzugsweise $CH_3$, —CN oder Cl, insbesondere bevorzugt —CN oder Cl.

R ist vorzugsweise geradkettiges Alkyl mit 1 bis 10, insbesondere mit 1 bis 7 C-Atomen, worin gegebenenfalls die mit dem asymmetrischen C-Atom verknüpfte $CH_2$-Gruppe durch —O—, —O—CO— oder —CO—O— ersetzt sein kann.

Vorzugsweise ist $R^2$ in Formel IV der optisch aktive Rest. Besonders bevorzugte optisch aktive Reste entsprechen hier der Formel

```
         *
—X—Q—CH—R
        |
        Y
```

worin X —O—, —CO—O— oder —O—CO—, Q —$CH_2$— oder eine Einfachbindung, Y $CH_3$ und R geradkettiges Alkyl mit 1 bis 7 C-Atomen bedeutet, worin die mit den asymmetrischen C-Atomen verknüpfte $CH_2$-Gruppe durch —O—, —CO—O— oder —O—CO— ersetzt ist.

$R^1$ und $R^2$ sind vorzugsweise Alkyl- oder Alkoxygruppen mit jeweils 3 bis 10 C-Atomen. Sie sind vorzugsweise geradkettig. Ferner bevorzugt sind jedoch Verbindungen der Formel IV, worin eine der Gruppen $R^1$ und $R^2$ eine verzweigte Alkyl- oder Alkoxygruppe bedeutet.

Pyr ist vorzugsweise in 2-Position mit Phe verknüpft.

$Z^1$ ist vorzugsweise —O—CO—.

m ist vorzugsweise 0.

Die lateral substituierte Phenylengruppe ist vorzugsweise eine durch ein F-Atom (= Phe(F)) oder durch eine CN-Gruppe substituierte 1,4-Phenylengruppe (= Phe(CN)).

Ferner bevorzugt sind Verbindungen mit einer 2,3-Dicyan-1,4-phenylengruppe (= $Phe(CN)_2$), insbesondere Derivate des 2,3-Dicyanhydrochinons (= Phe(2-CN, 3-CN)).

—$A^1$— ist ein Strukturelement ausgewählt aus der Gruppe der Formeln 1 bis 4:

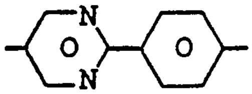, 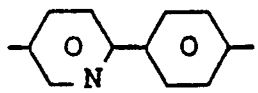, 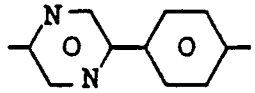, 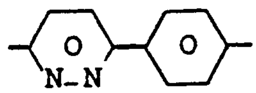

1 2 3 . 4

Gruppen der Formeln 1 und 2 sind besonders bevorzugt.

Unter den Verbindungen der Formel IV sowie der vor- und nachstehenden Teilformeln sind diejenigen bevorzugt, in denen mindestens einer der darin enthaltenen Reste eine der angegebenen bevorzugten Bedeutungen hat. Besonders bevorzugte kleinere Gruppen von Verbindungen sind diejenigen der Formeln IV1 bis IV12:

| | |
|---|---|
| $R^1$—pyr—Phe—OCO—Phe(F)—$R^2$ | IV1 |
| $R^1$—Pyr—Phe—COO—Phe(F)—$R^2$ | IV2 |
| $R^1$—Pyr—Phe—OCO—Phe(CN)—$R^2$ | IV3 |
| $R^1$—Pyr—Phe—COO—Phe(CN)—$R^2$ | IV4 |
| $R^1$—Phe—Pyr—OCO—Phe(F)—$R^2$ | IV5 |
| $R^1$—Phe—Pyr—COO—Phe(F)—$R^2$ | IV6 |
| $R^1$—Phe—Pyr—OCO—Phe(CN)—$R^2$ | IV7 |
| $R^1$—Phe—Pyr—COO—Phe(CN)—$R^2$ | IV8 |
| $R^1$—Pyr—Phe(F)—COO—$A^2$—$R^2$ | IV9 |
| $R^1$—Pyr—Phe(F)—OCO—$A^2$—$R^2$ | IV10 |
| $R^1$—Phe(F)—Pyr—OCO—$A^2$—$R^2$ | IV11 |
| $R^1$—Pyr—Phe—COO—Phe(Z—CN,3—CN)—$R^2$ | IV12 |

In den Teilformeln IV1, IV3, IV5 und IV12 bedeutet $R^2$ vorzugsweise Alkoxy mit 4 bis 10 C-Atomen.

Besonders bevorzugt sind die Verbindungen der Teilformeln IV1, IV2, IV4, IV9, IV10 und IV12, insbesondere bevorzugt diejenigen der Teilformeln IV1 und IV2.

Verbindungen der Formel IV, die keine $S_c$-Phasen aufweisen, eignen sich ebenfalls als Komponenten erfindungsgemäßer smektischer Phasen.

Alle Verbindungen der Formel IV werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten Umsetzung bekannt und geeignet sind. Dabei kann man auch von an sich bekannten, hier nicht näher erwähnten Varianten Gebrauch machen.

Die Verbindungen der Formel IV können beispielsweise durch Veresterung einer entsprechenden Carbonsäure oder eines ihrer reactionsfähigen Derivate mit einem entsprechenden Alkohol oder einem seiner reaktionsfähigen Derivate durch Umsetzung eines entsprechenden Amidins oder eines seiner Derivate mit einem 2-Acetyl-alkanaldialkylacetal oder einem seiner reaktionsfähigen Derivate, durch Ersatz der Diazoniumgruppe in einem entsprechenden Diazoniumsalz gegen Cl, F oder CN oder durch Umsetzung einer entsprechenden Chlor- oder Bromverbindung mit einem Cyanid hergestellt werden.

Die ferroelektrischen flüssigkristallinen Phasen bestehen aus 2 bis 15, vorzugsweise 3 bis 12 Komponenten, darunter mindestens einer Verbindung der Formel IV. Die anderen Bestandteile werden vorzugsweise ausgewählt aus den Verbindungen der Formeln II bis IV,

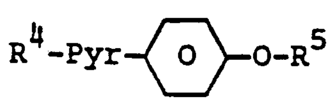

II

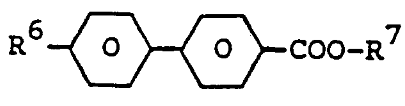

III

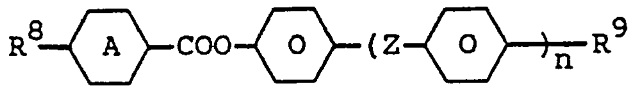

IV

worin $R^4$ und $R^5$ jeweils unabhängig voneinander n-Alkyl mit 5 bis 12 C-Atomen bedeutet und $R^6$, $R^7$, $R^8$ und $R^9$ jeweils unabhängig voneinander geradkettige oder verzweigte, gegebenenfalls chirale, Alkyl--, Alkoxy-, Alkoxycarbonyl- oder Alkanoyloxygruppen mit 5 bis 12, insbesondere mit 6 bis 10 C-Atomen bedeutet. Ring A ist 1,4-Phenylen oder trans-1,4-Cyclohexylen. n ist 0 oder 1.

Alle diese Substanzen sind nach literaturbekannten Methoden herstellbar.

Weiterhin bevorzugt sind ferroelektrische Phasen enthaltend mindestens eine Verbindung der Formel V

$$R^1—Q^1—A—(Q^2)_q—R^2 \qquad V$$

worin

$R^1$ und $R^2$ jeweils unabhängig voneinander eine geradkettige Alkylgruppe mit 1 bis 15 C-Atomen, worin auch eine oder mehrere nicht benachbarte $CH_2$-Gruppen durch —O—, —S—, —CO—, $CHCH_3$—O—, —$CHCH_3$—, —CH-Halogen—, CHCN—, —O—CO—, —O—COO—, —CO—O— und/oder —CH=CH— ersetzt sein können,

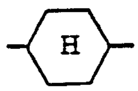, 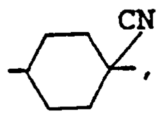, 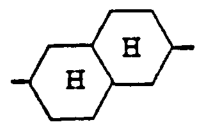, 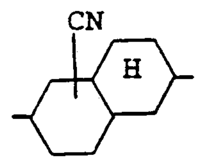, 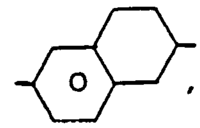,

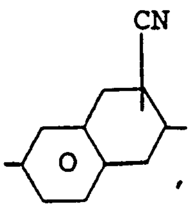, 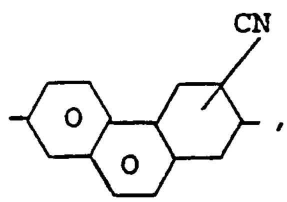, 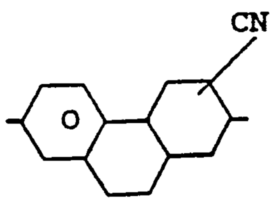, 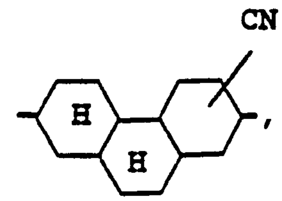,

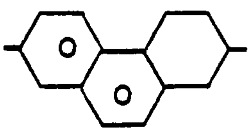, 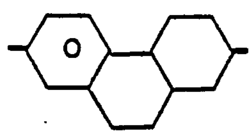, 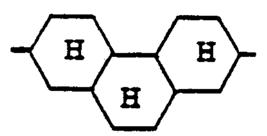, 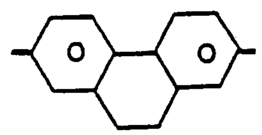,

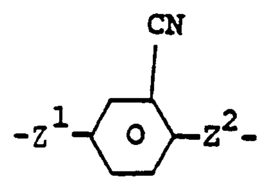 oder 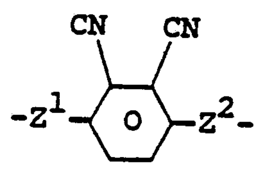

q 0 oder 1,

$Q^1$ und $Q^2$ jeweils unabhängig voneinander, $—(A°—Z°)_p—$, wobei

A° unsubstituiertes oder ein- oder mehrfach durch Halogenatome, $CH_3$- und/oder Nitril-Gruppen substituiertes 1,4-Cyclohexylen, worin auch eine oder zwei nicht benachbarte $CH_2$-Gruppen durch —O— und/oder —S— und/oder eine

$$\overset{|}{—CH}—CH_2—$$

Gruppierung durch

$$\overset{|}{—C}=N—$$

ersetzt sein könn en (Cy), oder unsubstituiertes oder ein- oder mehrfach durch Halogenatome, $CH_3$- und/ oder Nitril-Gruppen substituiertes 1,4-Phenylen, worin auch eine oder mehrere CH-Gruppen durch N ersetzt sein können (Ph) bedeutet, einer der Reste A° auch 2,6-Naphthylen (Na) oder Tetrahydro-2,6-naphthylen (4H-Na), gegebenenfalls durch Halogen oder CN substituiert,

$Z^{\circ}$, $Z^1$ und $Z^2$ jeweils unabhängig voneinander —CO—O—, —O—CO—, —$CH_2O$—, $OCH_2$—, —$CH_2CH_2$—, —$CHCNCH_2$—, —$CH_2$—CHCN— oder eine Einfachbindung, und

p 1, 2 oder 3, oder im Falle A = Tetra- oder Octahydrophenanthren auch 0 bedeutet, wobei im Falle A =

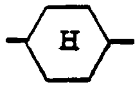, 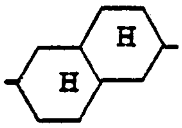, 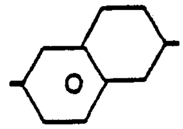, 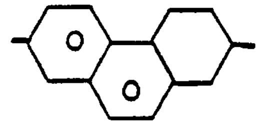, 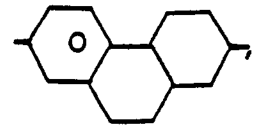

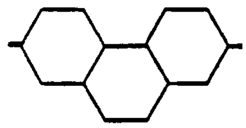, oder 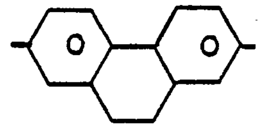

mindestens eine Gruppe $Z^{\circ}$ —$CHCNCH_2$— oder —$CH_2CHCN$— bedeutet und/oder in mindestens einer der Gruppe $R^1$ und $R^2$ mindestens eine $CH_2$-Gruppe durch —CHCN— ersetzt ist.

Die Verbindungen der Formel V können geradkettige oder verzweigte Flügelgruppen $R^1$ und/oder $R^2$ haen. Verbindungen mit verzweigten Flügelgruppen können in Form des Racemates oder als optisch aktive Verbindungen eingesetzt werden. Achirale Basismischungen aus Verbindungen der Formel V und gegebenenfalls weiteren achiralen Komponenten können mit chiralen Vebrindungen der Formel I der auch zusätzlich mit anderen chiralen Verbindungen dotiert werden, um chiral getiltete smektische Phasen zu erhalten.

Besonders bevorzugte kleinere Gruppen von Verbindungen sind diejenigen der Formeln V1 bis V18:

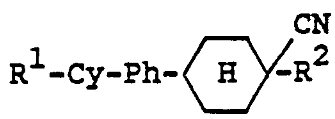 V1

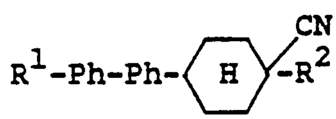 V2

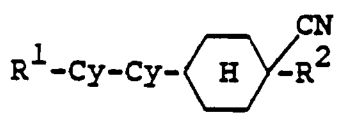 V3

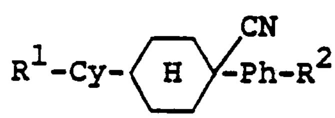 V4

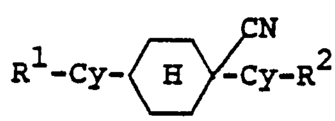 V5

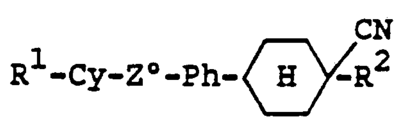 V6

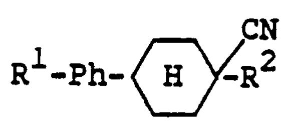 V7

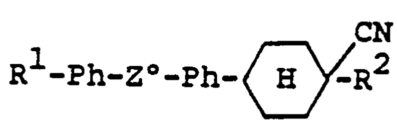 V8

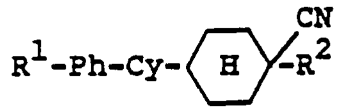 V9

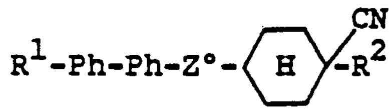 V10

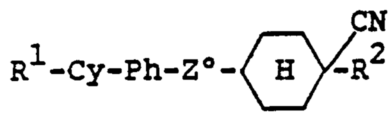 V11

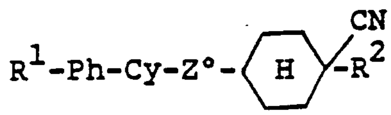 V12

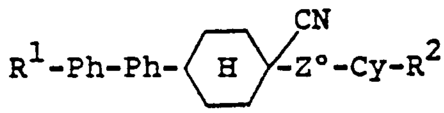 V13

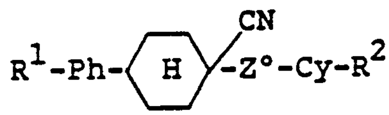 V14

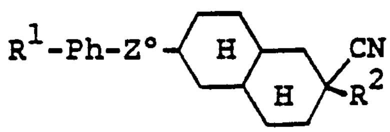 V15

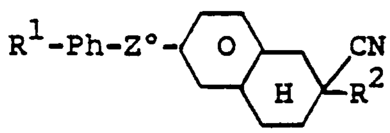 V16

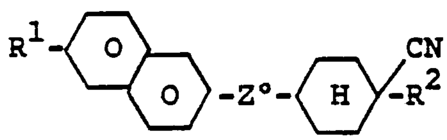 V17

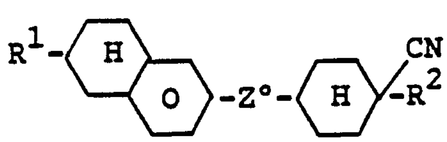 V18

Eine weitere besonders bevorzugte kleinere Gruppe von Verbindungen sind diejenigen der Formeln V19 bis V22:

$$R^1—A°—Cy—(CH_2)_r—CHCN—C_sH_{2s}$$ V19

$$R^1—A°—A°—Cy—(CH_2)_r—CHCN—C_sH_{2s+1}$$ V20

$$R^1—A°—A°—CHCN—CH_2—Cy—R^2$$ V21

$$R^1—A°—A°—CH_2—CHCN—Cy—R^2$$ V22

worin r 0, 1, 2 oder 3 bedeutet und (r + s) 1 bis 14 ist.

Verbindungen der Formel V, die keine $S_c$-Phasen aufweisen eignen sich ebenfalls als Komponenten erfindungsgemäßer smecktischer Phasen.

Alle Verbindungen der Formel V werden nach an sich bekannten Methoden hergestellt, wie sie in der Literatur (z.B. in den Standardwerken wie Houben-Weyl, Methoden der Organischen Chemie, Georg-Thieme-Verlag, Stuttgart) beschrieben sind, und zwar unter Reaktionsbedingungen, die für die genannten

Umsetzungen bekannt und geeignet sind. Dabei kann man auch von an sich bekannten, hier nicht näher erwähnten Varianten Gebrauch machen.

Formel V umfaßt zum überwiegenden Teil bekannte Verbindungen, wie beispielsweise die bevorzugten, in DOS 32 31 707, 33 19 781, 33 20 024, 34 07 013, 34 43 029, 33 32 690, 33 32 691, 33 32 692, 29 33 563, 28 53 728, 26 13 293, 34 01 320, 31 36 624, 30 40 632, 32 05 766, 22 40 864, 29 37 700, 34 10 734, 33 24 686, EP—OS 0 085 995, EP—OS 0 084 194, DD 116 732, FR 24 25 469, FR 24 19 966, USP 4 237 026, USP 3 953 491, USP 4 225 454 bzw. in H. J. Deutscher et al., J. prakt Chemie, 321, 569 (1979) und J. C. Dubois et al., Mol. Cryst. Liq. Cryst. *47*, 193 (1978) beschriebenen Verbindungen.

Als Komponenten der Phasen kommen ferner Verbindungen der Formel

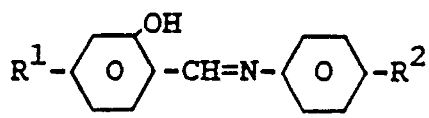

in Frage, worin $R^1$ und $R^2$ die bei Formel V angegebene Bedeutung haben.

Besonders bevorzugt sind chiral getiltete smektische flüssigkristalline Phasen, deren achirale Basismischung neben Verbindungen der Formel IV mindestens eine andere Komponente mit negativer oder betragsmäßig kleiner positiver dielektrischer Anisotropie enthält. Als weiter Komponenten mit betragsmäßig kleiner positiver oder negativer dielektrischer Anisotropie eignen sich Verbindungen der Teilformeln Va bis Vp:

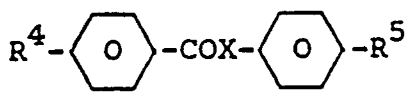 Va

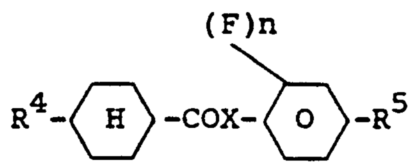 Vb

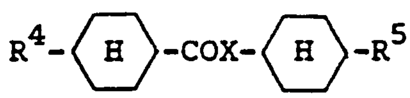 Vc

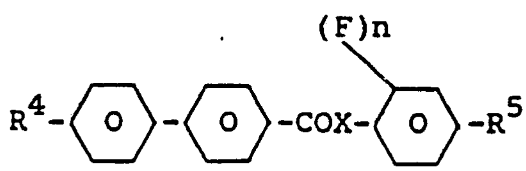 Vd

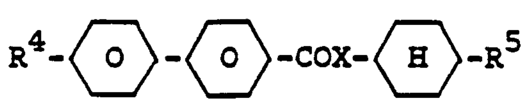 Ve

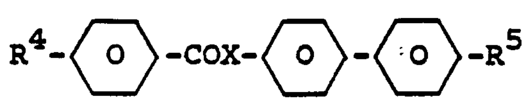 Vf

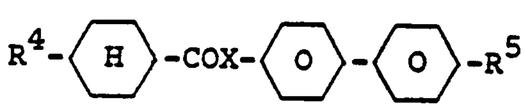 Vg

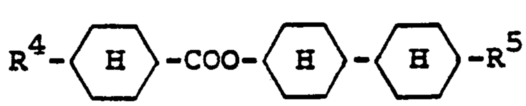 Vh

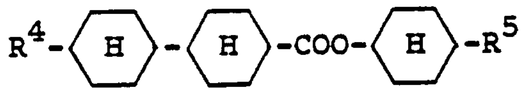

Vi

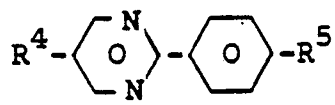

Vj

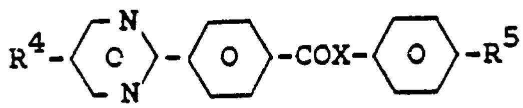

Vk

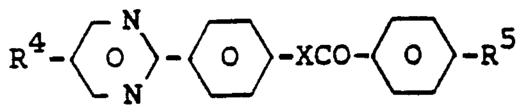

Vl

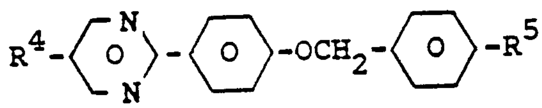

Vm

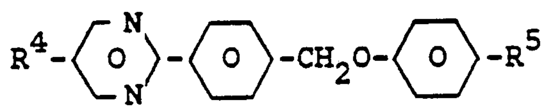

Vn

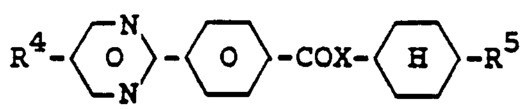

Vo

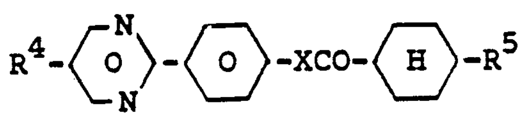

Vp

$R^4$ und $R^5$ sind jeweils vorzugsweise geradkettiges Alkyl, Alkoxy, Alkanoyloxy oder Alkoxycarbonyl mit jeweils 3 bis 12 C-Atomen. X ist vorzugsweise O. n ist 0 oder 1.

Besonders bevorzugt sind die Verbindungen der Teilformeln Va, Vb, Vd und Vf, worin $R^4$ und $R^5$ jeweils geradkettiges Alkyl oder Alkoxy mit jeweils 5 bis 10 C-Atomen bedeutet.

Die Verbindungen der Teilformeln Vc, Vh und Vi eignen sich als Zusätze zur Schmelzpunkterniedrigung und weden normalerweise den Basismischungen mit nicht mehr als 5%, vorzugsweise 1 bis 3% zugesetzt. $R^4$ und $R^5$ bedeuten in den Verbindungen der Teilformeln Vc, Vh und Vi vorzugsweise geradkettiges Alkyl mit 2 bis 7, vorzugsweise 3 bis 5, C-Atomen. Eine weitere zur Schmelzpunktserniedrigung in den erfindungsgemäßen Phasen geeignete Verbindungsklasse ist. diejenige der Formel

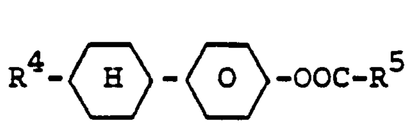

worin $R^4$ und $R^5$ die für Vc, Vh und Vi angegebene bevorzugte Bedeutung haben.

Als weitere Komponenten mit negativer dielektrischer Anisotropie eignen sich weiterhin Verbindungen enthaltend das Strukturelement B oder C.

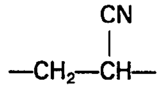

B

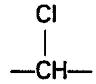

C

Bevorzugte Verbindungen dieser Art entsprechen den Formeln VIb und VIc:

$$R'\text{—}Q^1\text{—}CH_2\text{—}\underset{\displaystyle CN}{\underset{|}{CH}}\text{—}Q^2\text{—}R'' \qquad \text{VIb}$$

$$R'\text{—}Q^3\text{—}Q^4\text{—}R''' \qquad \text{VIc}$$

R' und R'' bedeuten jeweils vorzugsweise geradkettige Alkyl- oder Alkox-Gruppen mit jeweils 2 bis 10 C-Atomen. $Q^1$ und $Q^2$ bedeuten jeweils 1,4-Phenylen, trans-1,4-Cyclohexylen, 4,4'-Biphenylyl, 4-(trans-4-Cyclohexyl)-phenyl, trans,trans-4,4'-Bicyclohexyl oder eine der Gruppen $Q^1$ und $Q^2$ auch eine Einfachbindung.

$Q^3$ und $Q^4$ bedeuten jeweils 1,4-Phenylen, 4,4'-Biphenylyl oder trans-1,4-Cyclohexylen. Eine der Gruppen $Q^3$ und $Q^4$ kann auch 1,4-Phenylen bedeuten, worin mindestens eine CH-Gruppe durch N ersetzt ist. R''' ist ein optisch aktiver Rest mit einem asymmetrischen Kohlenstoffatom der Struktur

$$\text{—}\overset{\displaystyle Cl}{\overset{|}{CH^*}}\text{—} \quad \text{oder} \quad \text{—}\overset{\displaystyle CN}{\overset{|}{CH^*}}\text{—}.$$

Besonders bevorzugte Verbindungen der Formel VIc sind diejenigen der Formel VIc':

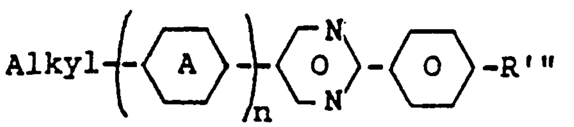

VIC'

worin A 1,4-Phenylen oder trans-1,4-Cyclohexylen und n 0 oder 1 bedeutet.

Die Phasen enthalten etwa 0,5—40%, vorzugsweise 5—10%, einer oder mehrerer Verbindungen der Formel I.

Weiterhin bevorzugt sind Phasen enthaltend 0,3—5%, vorzugsweise 1—4%, einer oder mehrerer Verbindungen der Formel I.

Die Herstellung der Dielektrika erfolgt in an sich üblicher Weise. In der Regel werden die Komponenten ineinander gelöst, zweckmäßig bei erhöhter Temperatur.

Durch geeignete Zusätze können die flüssigkristallinen Phasen so modifiziert werden, daß sie in allen bisher bekannt gewordenen Arten von Flüssigkristallanzeigeelementen verwendet werden können.

Die folgenden Biespiele sollen die Erfindung erläutern, ohne sie zu begrenzen. F. = Schmelzpunkt, K. = Klärpunkt. Vor- und nachstehend bedeuten Prozentangaben Gewichtsprozent; alle Temperaturangaben sind in Grad Celsuis angegeben. Die Werte für die spontane Polarisation gelten für Raumtemperatur. Es bedeuten ferner: K. Kristallinfester Zustand, S: Smektische Phase (der Index kennzeichnet den Phasentyp), N: Nematischer Zustand, Ch: Cholesterische Phase, I: Isotrope Phase. Die zwischen zwei Symbolen stehende Zahl gibt die Umwandlungstemperaturen in Grad Celsius an. "Übliche Aufarbeitung" bedeutet: man gibt Wasser hinzu, extrahiert mit Methylenchlorid, trennt ab, trocknet die organische Phase, dampft ein und reinigt das produkt durch Kristallisation und/oder Chromatographie.

## Beispiel 1

30 g 2-Fluor-4-decyloxylbenzoesäure (hergestellt aus 2-Fluor-4-hydroxy-benzoesäure mit Decylbromid in DMF/Kaliumcarbonat) werden mit 15 g Thionylchlorid in 120 ml Toluol bis zum Ende der Chlorwasserstoffentwicklung am Rückfluß erhitzt und in das entspr. Benzoylchlorid überführt und mit einem Gemisch von 28,4 g 4-(5-Octylpyrimidin-2-yl)-phenol und 10 ml Triethylamin versetzt. Nach 20 Stunden Rühren bei 20° filtriert man ausgeschiedenes Triethylammoniumchlorid ab und engt das Filtrat ein. Es kristallisiert 2-Fluor-4-decyloxybenzoesäure-(4-(5-octylpyrimidin-2-yl)-phenyl)-ester.

Analog dazu werden erhalten:

2-Fluor-4-butyloxybenzoesäure-(4-(5-propylpyrimidin-2-yl)-phenyl-ester
2-Fluor-4-butyloxybenzoesäure-(4-(5-pentylpyrimidin-2-yl)-phenyl-ester
2-Fluor-4-butyloxybenzoesäure-(4-(5-hexylpyrimidin-2-yl)-phenyl-ester
2-Fluor-4-butyloxybenzoesäure-(4-(5-heptylpyrimidin-2-yl)-phenyl-ester
2-Fluor-4-butyloxybenzoesäure-(4-(5-octylpyrimidin-2-yl)-phenyl-ester
2-Fluor-4-butyloxybenzoesäure-(4-(5-nonylpyrimidin-2-yl)-phenyl-ester
2-Fluor-4-butyloxybenzoesäure-(4-(5-decylpyrimidin-2-yl)-phenyl-ester
2-Fluor-4-pentyloxybenzoesäure-(4-(5-propylpyrimidin-2-yl)-phenyl-ester
2-Fluor-4-pentyloxybenzoesäure-(4-(5-pentylpyrimidin-2-yl)-phenyl-ester
2-Fluor-4-pentyloxybenzoesäure-(4-(5-hexylpyrimidin-2-yl)-phenyl-ester
2-Fluor-4-pentyloxybenzoesäure-(4-(5-heptylpyrimidin-2-yl)-phenyl-ester
2-Fluor-4-pentyloxybenzoesäure-(4-(5-octylpyrimidin-2-yl)-phenyl-ester
2-Fluor-4-pentyloxybenzoesäure-(4-(5-nonylpyrimidin-2-yl)-phenyl-ester

2-Fluor-4-pentyloxybenzoesäure-(4-(5-decylpyrimidin-2-yl)-phenyl-ester

2-Fluor-4-hexyloxybenzoesäure-(4-(5-propylpyrimidin-2-yl)-phenyl-ester
2-Fluor-4-hexyloxybenzoesäure-(4-(5-pentylpyrimidin-2-yl)-phenyl-ester
2-Fluor-4-hexyloxybenzoesäure-(4-(5-hexylpyrimidin-2-yl)-phenyl-ester
2-Fluor-4-hexyloxybenzoesäure-(4-(5-heptylpyrimidin-2-yl)-phenyl-ester
2-Fluor-4-hexyloxybenzoesäure-(4-(5-octylpyrimidin-2-yl)-phenyl-ester
2-Fluor-4-hexyloxybenzoesäure-(4-(5-nonylpyrimidin-2-yl)-phenyl-ester
2-Fluor-4-hexyloxybenzoesäure-(4-(5-decylpyrimidin-2-yl)-phenyl-ester
2-Fluor-4-heptyloxybenzoesäure-(4-(5-propylpyrimidin-2-yl)-phenyl-ester
2-Fluor-4-heptyloxybenzoesäure-(4-(5-pentylpyrimidin-2-yl)-phenyl-ester
2-Fluor-4-heptyloxybenzoesäure-(4-(5-hexylpyrimidin-2-yl)-phenyl-ester
2-Fluor-4-heptyloxybenzoesäure-(4-(5-heptylpyrimidin-2-yl)-phenyl-ester
2-Fluor-4-heptyloxybenzoesäure-(4-(5-octylpyrimidin-2-yl)-phenyl-ester
2-Fluor-4-heptyloxybenzoesäure-(4-(5-nonylpyrimidin-2-yl)-phenyl-ester
2-Fluor-4-heptyloxybenzoesäure-(4-(5-decylpyrimidin-2-yl)-phenyl-ester

2-Fluor-4-octyloxybenzoesäure-(4-(5-propylpyrimidin-2-yl)-phenyl-ester
2-Fluor-4-octyloxybenzoesäure-(4-(5-pentylpyrimidin-2-yl)-phenyl-ester
2-Fluor-4-octyloxybenzoesäure-(4-(5-hexylpyrimidin-2-yl)-phenyl-ester
2-Fluor-4-octyloxybenzoesäure-(4-(5-heptylpyrimidin-2-yl)-phenyl-ester
2-Fluor-4-octyloxybenzoesäure-(4-(5-octylpyrimidin-2-yl)-phenyl-ester
2-Fluor-4-octyloxybenzoesäure-(4-(5-nonylpyrimidin-2-yl)-phenyl-ester

2-Fluor-4-octyloxybenzoesäure-(4-(5-decylpyrimidin-2-yl)-phenyl-ester
2-Fluor-4-nonyloxybenzoesäure-(4-(5-propylpyrimidin-2-yl)-phenyl-ester
2-Fluor-4-nonyloxybenzoesäure-(4-(5-pentylpyrimidin-2-yl)-phenyl-ester
2-Fluor-4-nonyloxybenzoesäure-(4-(5-hexylpyrimidin-2-yl)-phenyl-ester
2-Fluor-4-nonyloxybenzoesäure-(4-(5-heptylpyrimidin-2-yl)-phenyl-ester
2-Fluor-4-nonyloxybenzoesäure-(4-(5-octylpyrimidin-2-yl)-phenyl-ester
2-Fluor-4-nonyloxybenzoesäure-(4-(5-nonylpyrimidin-2-yl)-phenyl-ester
2-Fluor-4-nonyloxybenzoesäure-(4-(5-decylpyrimidin-2-yl)-phenyl-ester
2-Fluor-4-decyloxybenzoesäure-(4-(5-propylpyrimidin-2-yl)-phenyl-ester
2-Fluor-4-decyloxybenzoesäure-(4-(5-pentylpyrimidin-2-yl)-phenyl-ester
2-Fluor-4-decyloxybenzoesäure-(4-(5-hexylpyrimidin-2-yl)-phenyl-ester
2-Fluor-4-decyloxybenzoesäure-(4-(5-heptylpyrimidin-2-yl)-phenyl-ester
2-Fluor-4-decyloxybenzoesäure-(4-(5-octylpyrimidin-2-yl)-phenyl-ester
2-Fluor-4-decyloxybenzoesäure-(4-(5-nonylpyrimidin-2-yl)-phenyl-ester
2-Fluor-4-decyloxybenzoesäure-(4-(5-decylpyrimidin-2-yl)-phenyl-ester
3-Fluor-4-heptyloxybenzoesäure-(4-(5-propylpyrimidin-2-yl)-phenyl-ester

3-Fluor-4-heptyloxybenzoesäure-(4-(5-pentylpyrimidin-2-yl)-phenyl-ester
3-Fluor-4-heptyloxybenzoesäure-(4-(5-hexylpyrimidin-2-yl)-phenyl-ester
3-Fluor-4-heptyloxybenzoesäure-(4-(5-heptylpyrimidin-2-yl)-phenyl-ester
3-Fluor-4-heptyloxybenzoesäure-(4-(5-octylpyrimidin-2-yl)-phenyl-ester
3-Fluor-4-heptyloxybenzoesäure-(4-(5-nonylpyrimidin-2-yl)-phenyl-ester, K 93° $S_c$ (88°) N 147° I
3-Fluor-4-heptyloxybenzoesäure-(4-(5-decylpyrimidin-2-yl)-phenyl-ester
3-Fluor-4-octyloxybenzoesäure-(4-(5-propylpyrimidin-2-yl)-phenyl-ester
3-Fluor-4-octyloxybenzoesäure-(4-(5-pentylpyrimidin-2-yl)-phenyl-ester
3-Fluor-4-octyloxybenzoesäure-(4-(5-hexylpyrimidin-2-yl)-phenyl-ester
3-Fluor-4-octyloxybenzoesäure-(4-(5-heptylpyrimidin-2-yl)-phenyl-ester
3-Fluor-4-octyloxybenzoesäure-(4-(5-octylpyrimidin-2-yl)-phenyl-ester
3-Fluor-4-octyloxybenzoesäure-(4-(5-nonylpyrimidin-2-yl)-phenyl-ester
3-Fluor-4-octyloxybenzoesäure-(4-(5-decylpyrimidin-2-yl)-phenyl-ester

3-Fluor-4-nonyloxybenzoesäure-(4-(5-propylpyrimidin-2-yl)-phenyl-ester
3-Fluor-4-nonyloxybenzoesäure-(4-(5-pentylpyrimidin-2-yl)-phenyl-ester
3-Fluor-4-nonyloxybenzoesäure-(4-(5-hexylpyrimidin-2-yl)-phenyl-ester
3-Fluor-4-nonyloxybenzoesäure-(4-(5-heptylpyrimidin-2-yl)-phenyl-ester
3-Fluor-4-nonyloxybenzoesäure-(4-(5-octylpyrimidin-2-yl)-phenyl-ester
3-Fluor-4-nonyloxybenzoesäure-(4-(5-nonylpyrimidin-2-yl)-phenyl-ester

3-Fluor-4-nonyloxybenzoesäure-(4-(5-decylpyrimidin-2-yl)-phenyl-ester
3-Chlor-4-hexyloxybenzoesäure-(4-(5-propylpyrimidin-2-yl)-phenyl-ester
3-Chlor-4-hexyloxybenzoesäure-(4-(5-pentylpyrimidin-2-yl)-phenyl-ester
3-Chlor-4-hexyloxybenzoesäure-(4-(5-hexylpyrimidin-2-yl)-phenyl-ester
3-Chlor-4-hexyloxybenzoesäure-(4-(5-heptylpyrimidin-2-yl)-phenyl-ester
3-Chlor-4-hexyloxybenzoesäure-(4-(5-octylpyrimidin-2-yl)-phenyl-ester
3-Chlor-4-hexyloxybenzoesäure-(4-(5-nonylpyrimidin-2-yl)-phenyl-ester
3-Chlor-4-hexyloxybenzoesäure-(4-(5-decylpyrimidin-2-yl)-phenyl-ester
3-Chlor-4-heptyloxybenzoesäure-(4-(5-propylpyrimidin-2-yl)-phenyl-ester
3-Chlor-4-heptyloxybenzoesäure-(4-(5-pentylpyrimidin-2-yl)-phenyl-ester
3-Chlor-4-heptyloxybenzoesäure-(4-(5-hexylpyrimidin-2-yl)-phenyl-ester
3-Chlor-4-heptyloxybenzoesäure-(4-(5-heptylpyrimidin-2-yl)-phenyl-ester
3-Chlor-4-heptyloxybenzoesäure-(4-(5-octylpyrimidin-2-yl)-phenyl-ester
3-Chlor-4-heptyloxybenzoesäure-(4-(5-nonylpyrimidin-2-yl)-phenyl-ester
3-Chlor-4-heptyloxybenzoesäure-(4-(5-decypyrimidin-2-yl)-phenyl-ester
3-Chlor-4-octyloxybenzoesäure-(4-(5-propylpyrimidin-2-yl)-phenyl-ester
3-Chlor-4-octyloxybenzoesäure-(4-(5-pentylpyrimidin-2-yl)-phenyl-ester
3-Chlor-4-octyloxybenzoesäure-(4-(5-hexylpyrimidin-2-yl)-phenyl-ester
3-Chlor-4-octyloxybenzoesäure-(4-(5-heptylpyrimidin-2-yl)-phenyl-ester
3-Chlor-4-octyloxybenzoesäure-(4-(5-octylpyrimidin-2-yl)-phenyl-ester
3-Chlor-4-octyloxybenzoesäure-(4-(5-nonylpyrimidin-2-yl)-phenyl-ester
3-Chlor-4-octyloxybenzoesäure-(4-(5-decylpyrimidin-2-yl)-phenyl-ester
3-Chlor-4-(2-octyloxy)-benzoesäure-(4-(5-propylpyrimidin-2-yl)-phenyl-ester
3-Chlor-4-(2-octyloxy)-benzoesäure-(4-(5-pentylpyrimidin-2-yl)-phenyl-ester
3-Chlor-4-(2-octyloxy)-benzoesäure-(4-(5-hexylpyrimidin-2-yl)-phenyl-ester
3-Chlor-4-(2-octyloxy)-benzoesäure-(4-(5-heptylpyrimidin-2-yl)-phenyl-ester
3-Chlor-4-(2-octyloxy)-benzoesäure-(4-(5-octylpyrimidin-2-yl)-phenyl-ester
3-Chlor-4-(2-octyloxy)-benzoesäure-(4-(5-nonylpyrimidin-2-yl)-phenyl-ester, K 40° $S_c$ × 42° CH 56° I, $P_s$ = 72 nC/cm$^2$ bei 25° im unterkühlten Zustand,
3-Chlor-4-(2-octyloxy)-benzoesäure-(4-(5-decylpyrimidin-2-yl)-phenyl-ester
3-Chlor-4-nonyloxybenzoesäure-(4-(5-propylpyrimidin-2-yl)-phenyl-ester
3-Chlor-4-nonyloxybenzoesäure-(4-(5-pentylpyrimidin-2-yl)-phenyl-ester
3-Chlor-4-nonyloxybenzoesäure-(4-(5-hexylpyrimidin-2-yl)-phenyl-ester
3-Chlor-4-nonyloxybenzoesäure-(4-(5-heptylpyrimidin-2-yl)-phenyl-ester
3-Chlor-4-nonyloxybenzoesäure-(4-(5-octylpyrimidin-2-yl)-phenyl-ester
3-Chlor-4-nonyloxybenzoesäure-(4-(5-nonylpyrimidin-2-yl)-phenyl-ester
3-Chlor-4-nonyloxybenzoesäure-(4-(5-decylpyrimidin-2-yl)-phenyl-ester
3-Brom-4-hexyloxybenzoesäure-(4-(5-propylpyrimidin-2-yl)-phenyl-ester
3-Brom-4-hexyloxybenzoesäure-(4-(5-pentylpyrimidin-2-yl)-phenyl-ester
3-Brom-4-hexyloxybenzoesäure-(4-(5-hexylpyrimidin-2-yl)-phenyl-ester
3-Brom-4-hexyloxybenzoesäure-(4-(5-heptylpyrimidin-2-yl)-phenyl-ester
3-Brom-4-hexyloxybenzoesäure-(4-(5-octylpyrimidin-2-yl)-phenyl-ester
3-Brom-4-hexyloxybenzoesäure-(4-(5-nonylpyrimidin-2-yl)-phenyl-ester
3-Brom-4-hexyloxybenzoesäure-(4-(5-decylpyrimidin-2-yl)-phenyl-ester
3-Brom-4-heptyloxybenzoesäure-(4-(5-propylpyrimidin-2-yl)-phenyl-ester
3-Brom-4-heptyloxybenzoesäure-(4-(5-pentylpyrimidin-2-yl)-phenyl-ester
3-Brom-4-heptyloxybenzoesäure-(4-(5-hexylpyrimidin-2-yl)-phenyl-ester
3-Brom-4-heptyloxybenzoesäure-(4-(5-heptylpyrimidin-2-yl)-phenyl-ester
3-Brom-4-heptyloxybenzoesäure-(4-(5-octylpyrimidin-2-yl)-phenyl-ester
3-Brom-4-heptyloxybenzoesäure-(4-(5-nonylpyrimidin-2-yl)-phenyl-ester
3-Brom-4-heptyloxybenzoesäure-(4-(5-decylpyrimidin-2-yl)-phenyl-ester
3-Brom-4-octyloxybenzoesäure-(4-(5-propylpyrimidin-2-yl)-phenyl-ester
3-Brom-4-octyloxybenzoesäure-(4-(5-pentylpyrimidin-2-yl)-phenyl-ester
3-Brom-4-octyloxybenzoesäure-(4-(5-hexylpyrimidin-2-yl)-phenyl-ester
3-Brom-4-octyloxybenzoesäure-(4-(5-heptylpyrimidin-2-yl)-phenyl-ester, K86° $S_c$ (79°) N 128° I
3-Brom-4-octyloxybenzoesäure-(4-(5-octylpyrimidin-2-yl)-phenyl-ester
3-Brom-4-octyloxybenzoesäure-(4-(5-nonylpyrimidin-2-yl)-phenyl-ester, K 80° $S_c$ 96° N 125° I
3-Brom-4-octyloxybenzoesäure-(4-(5-decylpyrimidin-2-yl)-phenyl-ester
3-Brom-4-(2-octyloxy)-benzoesäure-(4-(5-propylpyrimidin-2-yl)-phenyl-ester
3-Brom-4-(2-octyloxy)-benzoesäure-(4-(5-pentylpyrimidin-2-yl)-phenyl-ester
3-Brom-4-(2-octyloxy)-benzoesäure-(4-(5-hexylpyrimidin-2-yl)-phenyl-ester
3-Brom-4-(2-octyloxy)-benzoesäure-(4-(5-heptylpyrimidin-2-yl)-phenyl-ester
3-Brom-4-(2-octyloxy)-benzoesäure-(4-(5-octylpyrimidin-2-yl)-phenyl-ester
3-Brom-4-(2-octyloxy)-benzoesäure-(4-(5-nonylpyrimidin-2-yl)-phenyl-ester
3-Brom-4-(2-octyloxy)-benzoesäure-(4-(5-decylpyrimidin-2-yl)-phenyl-ester

3-Brom-4-nonyloxybenzoesäure-(4-(5-propylpyrimidin-2-yl)-phenyl-ester
3-Brom-4-nonyloxybenzoesäure-(4-(5-pentylpyrimidin-2-yl)-phenyl-ester
3-Brom-4-nonyloxybenzoesäure-(4-(5-hexylpyrimidin-2-yl)-phenyl-ester
3-Brom-4-nonyloxybenzoesäure-(4-(5-heptylpyrimidin-2-yl)-phenyl-ester
3-Brom-4-nonyloxybenzoesäure-(4-(5-octylpyrimidin-2-yl)-phenyl-ester
3-Brom-4-nonyloxybenzoesäure-(4-(5-nonylpyrimidin-2-yl)-phenyl-ester
3-Brom-4-nonyloxybenzoesäure-(4-(5-decylpyrimidin-2-yl)-phenyl-ester
3-Cyan-4-hexyloxybenzoesäure-(4-(5-propylpyrimidin-2-yl)-phenyl-ester
3-Cyan-4-hexyloxybenzoesäure-(4-(5-pentylpyrimidin-2-yl)-phenyl-ester
3-Cyan-4-hexyloxybenzoesäure-(4-(5-hexylpyrimidin-2-yl)-phenyl-ester
3-Cyan-4-hexyloxybenzoesäure-(4-(5-heptylpyrimidin-2-yl)-phenyl-ester
3-Cyan-4-hexyloxybenzoesäure-(4-(5-octylpyrimidin-2-yl)-phenyl-ester
3-Cyan-4-hexyloxybenzoesäure-(4-(5-nonylpyrimidin-2-yl)-phenyl-ester
3-Cyan-4-hexyloxybenzoesäure-(4-(5-decylpyrimidin-2-yl)-phenyl-ester
3-Cyan-4-heptyloxybenzoesäure-(4-(5-propylpyrimidin-2-yl)-phenyl-ester
3-Cyan-4-heptyloxybenzoesäure-(4-(5-pentylpyrimidin-2-yl)-phenyl-ester
3-Cyan-4-heptyloxybenzoesäure-(4-(5-hexylpyrimidin-2-yl)-phenyl-ester
3-Cyan-4-heptyloxybenzoesäure-(4-(5-heptylpyrimidin-2-yl)-phenyl-ester
3-Cyan-4-heptyloxybenzoesäure-(4-(5-octylpyrimidin-2-yl)-phenyl-ester
3-Cyan-4-heptyloxybenzoesäure-(4-(5-nonylpyrimidin-2-yl)-phenyl-ester
3-Cyan-4-heptyloxybenzoesäure-(4-(5-decylpyrimidin-2-yl)-phenyl-ester
3-Cyan-4-octyloxybenzoesäure-(4-(5-propylpyrimidin-2-yl)-phenyl-ester
3-Cyan-4-octyloxybenzoesäure-(4-(5-pentylpyrimidin-2-yl)-phenyl-ester
3-Cyan-4-octyloxybenzoesäure-(4-(5-hexylpyrimidin-2-yl)-phenyl-ester
3-Cyan-4-octyloxybenzoesäure-(4-(5-heptylpyrimidin-2-yl)-phenyl-ester
3-Cyan-4-octyloxybenzoesäure-(4-(5-octylpyrimidin-2-yl)-phenyl-ester
3-Cyan-4-octyloxybenzoesäure-(4-(5-nonylpyrimidin-2-yl)-phenyl-ester, K 66° $S_c$ 120° N 123° I
3-Cyan-4-octyloxybenzoesäure-(4-(5-decylpyrimidin-2-yl)-phenyl-ester
3-Cyan-4-(2-octyloxy)-benzoesäure-(4-(5-propylpyrimidin-2-yl)-phenyl-ester
3-Cyan-4-(2-octyloxy)-benzoesäure-(4-(5-pentylpyrimidin-2-yl)-phenyl-ester
3-Cyan-4-(2-octyloxy)-benzoesäure-(4-(5-hexylpyrimidin-2-yl)-phenyl-ester
3-Cyan-4-(2-octyloxy)-benzoesäure-(4-(5-heptylpyrimidin-2-yl)-phenyl-ester
3-Cyan-4-(2-octyloxy)-benzoesäure-(4-(5-octylpyrimidin-2-yl)-phenyl-ester
3-Cyan-4-(2-octyloxy)-benzoesäure-(4-(5-nonylpyrimidin-2-yl)-phenyl-ester
3-Cyan-4-(2-octyloxy)-benzoesäure-(4-(5-decylpyrimidin-2-yl)-phenyl-ester
3-Cyan-4-nonyloxybenzoesäure-(4-(5-propylpyrimidin-2-yl)-phenyl-ester
3-Cyan-4-nonyloxybenzoesäure-(4-(5-pentylpyrimidin-2-yl)-phenyl-ester
3-Cyan-4-nonyloxybenzoesäure-(4-(5-hexylpyrimidin-2-yl)-phenyl-ester
3-Cyan-4-nonyloxybenzoesäure-(4-(5-heptylpyrimidin-2-yl)-phenyl-ester
3-Cyan-4-nonyloxybenzoesäure-(4-(5-octylpyrimidin-2-yl)-phenyl-ester
3-Cyan-4-nonyloxybenzoesäure-(4-(5-nonylpyrimidin-2-yl)-phenyl-ester
3-Cyan-4-nonyloxybenzoesäure-(4-(5-decylpyrimidin-2-yl)-phenyl-ester
3-Fluor-4-heptylbenzoesäure-(4-(5-propylpyrimidin-2-yl)-phenyl-ester
3-Fluor-4-heptylbenzoesäure-(4-(5-pentylpyrimidin-2-yl)-phenyl-ester
3-Fluor-4-heptylbenzoesäure-(4-(5-hexylpyrimidin-2-yl)-phenyl-ester
3-Fluor-4-heptylbenzoesäure-(4-(5-heptylpyrimidin-2-yl)-phenyl-ester
3-Fluor-4-heptylbenzoesäure-(4-(5-octylpyrimidin-2-yl)-phenyl-ester
3-Fluor-4-heptylbenzoesäure-(4-(5-nonylpyrimidin-2-yl)-phenyl-ester
3-Fluor-4-heptylbenzoesäure-(4-(5-decylpyrimidin-2-yl)-phenyl-ester
3-Fluor-4-octylbenzoesäure-(4-(5-propylpyrimidin-2-yl)-phenyl-ester
3-Fluor-4-octylbenzoesäure-(4-(5-pentylpyrimidin-2-yl)-phenyl-ester
3-Fluor-4-octylbenzoesäure-(4-(5-hexylpyrimidin-2-yl)-phenyl-ester
3-Fluor-4-octylbenzoesäure-(4-(5-heptylpyrimidin-2-yl)-phenyl-ester
3-Fluor-4-octylbenzoesäure-(4-(5-octylpyrimidin-2-yl)-phenyl-ester
3-Fluor-4-octylbenzoesäure-(4-(5-nonylpyrimidin-2-yl)-phenyl-ester
3-Fluor-4-octylbenzoesäure-(4-(5-decylpyrimidin-2-yl)-phenyl-ester
3-Fluor-4-nonylbenzoesäure-(4-(5-propylpyrimidin-2-yl)-phenyl-ester
3-Fluor-4-nonylbenzoesäure-(4-(5-pentylpyrimidin-2-yl)-phenyl-ester
3-Fluor-4-nonylbenzoesäure-(4-(5-hexylpyrimidin-2-yl)-phenyl-ester
3-Fluor-4-nonylbenzoesäure-(4-(5-heptylpyrimidin-2-yl)-phenyl-ester
3-Fluor-4-nonylbenzoesäure-(4-(5-octylpyrimidin-2-yl)-phenyl-ester
3-Fluor-4-nonylbenzoesäure-(4-(5-nonylpyrimidin-2-yl)-phenyl-ester
3-Fluor-4-nonylbenzoesäure-(4-(5-decylpyrimidin-2-yl)-phenyl-ester
3-Chlor-4-hexylbenzoesäure-(4-(5-propylpyrimidin-2-yl)-phenyl-ester
3-Chlor-4-hexylbenzoesäure-(4-(5-pentylpyrimidin-2-yl)-phenyl-ester

3-Chlor-4-hexylbenzoesäure-(4-(5-hexylpyrimidin-2-yl)-phenyl-ester
3-Chlor-4-hexylbenzoesäure-(4-(5-heptylpyrimidin-2-yl)-phenyl-ester
3-Chlor-4-hexylbenzoesäure-(4-(5-octylpyrimidin-2-yl)-phenyl-ester
3-Chlor-4-hexylbenzoesäure-(4-(5-nonylpyrimidin-2-yl)-phenyl-ester
3-Chlor-4-hexylbenzoesäure-(4-(5-decylpyrimidin-2-yl)-phenyl-ester
3-Chlor-4-heptylbenzoesäure-(4-(5-propylpyrimidin-2-yl)-phenyl-ester
3-Chlor-4-heptylbenzoesäure-(4-(5-pentylpyrimidin-2-yl)-phenyl-ester
3-Chlor-4-heptylbenzoesäure-(4-(5-hexylpyrimidin-2-yl)-phenyl-ester
3-Chlor-4-heptylbenzoesäure-(4-(5-heptylpyrimidin-2-yl)-phenyl-ester
3-Chlor-4-heptylbenzoesäure-(4-(5-octylpyrimidin-2-yl)-phenyl-ester
3-Chlor-4-heptylbenzoesäure-(4-(5-nonylpyrimidin-2-yl)-phenyl-ester
3-Chlor-4-heptylbenzoesäure-(4-(5-decylpyrimidin-2-yl)-phenyl-ester
3-Chlor-4-octylbenzoesäure-(4-(5-propylpyrimidin-2-yl)-phenyl-ester
3-Chlor-4-octylbenzoesäure-(4-(5-pentylpyrimidin-2-yl)-phenyl-ester
3-Chlor-4-octylbenzoesäure-(4-(5-hexylpyrimidin-2-yl)-phenyl-ester
3-Chlor-4-octylbenzoesäure-(4-(5-heptylpyrimidin-2-yl)-phenyl-ester
3-Chlor-4-octylbenzoesäure-(4-(5-octylpyrimidin-2-yl)-phenyl-ester
3-Chlor-4-octylbenzoesäure-(4-(5-nonylpyrimidin-2-yl)-phenyl-ester
3-Chlor-4-octylbenzoesäure-(4-(5-decylpyrimidin-2-yl)-phenyl-ester
3-Chlor-4-nonylbenzoesäure-(4-(5-propylpyrimidin-2-yl)-phenyl-ester
3-Chlor-4-nonylbenzoesäure-(4-(5-pentylpyrimidin-2-yl)-phenyl-ester
3-Chlor-4-nonylbenzoesäure-(4-(5-hexylpyrimidin-2-yl)-phenyl-ester
3-Chlor-4-nonylbenzoesäure-(4-(5-heptylpyrimidin-2-yl)-phenyl-ester
3-Chlor-4-nonylbenzoesäure-(4-(5-octylpyrimidin-2-yl)-phenyl-ester
3-Chlor-4-nonylbenzoesäure-(4-(5-nonylpyrimidin-2-yl)-phenyl-ester
3-Chlor-4-nonylbenzoesäure-(4-(5-decylpyrimidin-2-yl)-phenyl-ester
3-Brom-4-hexylbenzoesäure-(4-(5-propylpyrimidin-2-yl)-phenyl-ester
3-Brom-4-hexylbenzoesäure-(4-(5-pentylpyrimidin-2-yl)-phenyl-ester
3-Brom-4-hexylbenzoesäure-(4-(5-hexylpyrimidin-2-yl)-phenyl-ester
3-Brom-4-hexylbenzoesäure-(4-(5-heptylpyrimidin-2-yl)-phenyl-ester
3-Brom-4-hexylbenzoesäure-(4-(5-octylpyrimidin-2-yl)-phenyl-ester
3-Brom-4-hexylbenzoesäure-(4-(5-nonylpyrimidin-2-yl)-phenyl-ester
3-Brom-4-hexylbenzoesäure-(4-(5-decylpyrimidin-2-yl)-phenyl-ester
3-Brom-4-heptylbenzoesäure-(4-(5-propylpyrimidin-2-yl)-phenyl-ester
3-Brom-4-heptylbenzoesäure-(4-(5-pentylpyrimidin-2-yl)-phenyl-ester
3-Brom-4-heptylbenzoesäure-(4-(5-hexylpyrimidin-2-yl)-phenyl-ester
3-Brom-4-heptylbenzoesäure-(4-(5-heptylpyrimidin-2-yl)-phenyl-ester
3-Brom-4-heptylbenzoesäure-(4-(5-octylpyrimidin-2-yl)-phenyl-ester
3-Brom-4-heptylbenzoesäure-(4-(5-nonylpyrimidin-2-yl)-phenyl-ester
3-Brom-4-heptylbenzoesäure-(4-(5-decylpyrimidin-2-yl)-phenyl-ester
3-Brom-4-octylbenzoesäure-(4-(5-propylpyrimidin-2-yl)-phenyl-ester
3-Brom-4-octylbenzoesäure-(4-(5-pentylpyrimidin-2-yl)-phenyl-ester
3-Brom-4-octylbenzoesäure-(4-(5-hexylpyrimidin-2-yl)-phenyl-ester, K 80° N 86° I
3-Brom-4-octylbenzoesäure-(4-(5-heptylpyrimidin-2-yl)-phenyl-ester, K 62° N 94° I
3-Brom-4-octylbenzoesäure-(4-(5-octylpyrimidin-2-yl)-phenyl-ester
3-Brom-4-octylbenzoesäure-(4-(5-nonylpyrimidin-2-yl)-phenyl-ester, K 51° $S_c$ 64° N 93° I
3-Brom-4-octylbenzoesäure-(4-(5-decylpyrimidin-2-yl)-phenyl-ester
3-Brom-4-nonylbenzoesäure-(4-(5-propylpyrimidin-2-yl)-phenyl-ester
3-Brom-4-nonylbenzoesäure-(4-(5-pentylpyrimidin-2-yl)-phenyl-ester
3-Brom-4-nonylbenzoesäure-(4-(5-hexylpyrimidin-2-yl)-phenyl-ester
3-Brom-4-nonylbenzoesäure-(4-(5-heptylpyrimidin-2-yl)-phenyl-ester
3-Brom-4-nonylbenzoesäure-(4-(5-octylpyrimidin-2-yl)-phenyl-ester
3-Brom-4-nonylbenzoesäure-(4-(5-nonylpyrimidin-2-yl)-phenyl-ester
3-Brom-4-nonylbenzoesäure-(4-(5-decylpyrimidin-2-yl)-phenyl-ester
3-Cyan-4-hexylbenzoesäure-(4-(5-propylpyrimidin-2-yl)-phenyl-ester
3-Cyan-4-hexylbenzoesäure-(4-(5-pentylpyrimidin-2-yl)-phenyl-ester
3-Cyan-4-hexylbenzoesäure-(4-(5-hexylpyrimidin-2-yl)-phenyl-ester
3-Cyan-4-hexylbenzoesäure-(4-(5-heptylpyrimidin-2-yl)-phenyl-ester
3-Cyan-4-hexylbenzoesäure-(4-(5-octylpyrimidin-2-yl)-phenyl-ester
3-Cyan-4-hexylbenzoesäure-(4-(5-nonylpyrimidin-2-yl)-phenyl-ester
3-Cyan-4-hexylbenzoesäure-(4-(5-decylpyrimidin-2-yl)-phenyl-ester
3-Cyan-4-heptylbenzoesäure-(4-(5-propylpyrimidin-2-yl)-phenyl-ester
3-Cyan-4-heptylbenzoesäure-(4-(5-pentylpyrimidin-2-yl)-phenyl-ester
3-Cyan-4-heptylbenzoesäure-(4-(5-hexylpyrimidin-2-yl)-phenyl-ester
3-Cyan-4-heptylbenzoesäure-(4-(5-heptylpyrimidin-2-yl)-phenyl-ester

3-Cyan-4-heptylbenzoesäure-(4-(5-octylpyrimidin-2-yl)-phenyl-ester
3-Cyan-4-heptylbenzoesäure-(4-(5-nonylpyrimidin-2-yl)-phenyl-ester
3-Cyan-4-heptylbenzoesäure-(4-(5-decylpyrimidin-2-yl)-phenyl-ester
3-Cyan-4-octylbenzoesäure-(4-(5-propylpyrimidin-2-yl)-phenyl-ester
3-Cyan-4-octylbenzoesäure-(4-(5-pentylpyrimidin-2-yl)-phenyl-ester
3-Cyan-4-octylbenzoesäure-(4-(5-hexylpyrimidin-2-yl)-phenyl-ester
3-Cyan-4-octylbenzoesäure-(4-(5-heptylpyrimidin-2-yl)-phenyl-ester
3-Cyan-4-octylbenzoesäure-(4-(5-octylpyrimidin-2-yl)-phenyl-ester
3-Cyan-4-octylbenzoesäure-(4-(5-nonylpyrimidin-2-yl)-phenyl-ester, K 88° $S_A$ (86°) N (87°) I
3-Cyan-4-octylbenzoesäure-(4-(5-decylpyrimidin-2-yl)-phenyl-ester
3-Cyan-4-nonylbenzoesäure-(4-(5-propylpyrimidin-2-yl)-phenyl-ester
3-Cyan-4-nonylbenzoesäure-(4-(5-pentylpyrimidin-2-yl)-phenyl-ester
3-Cyan-4-nonylbenzoesäure-(4-(5-hexylpyrimidin-2-yl)-phenyl-ester
3-Cyan-4-nonylbenzoesäure-(4-(5-heptylpyrimidin-2-yl)-phenyl-ester
3-Cyan-4-nonylbenzoesäure-(4-(5-octylpyrimidin-2-yl)-phenyl-ester
3-Cyan-4-nonylbenzoesäure-(4-(5-nonylpyrimidin-2-yl)-phenyl-ester
3-Cyan-4-nonylbenzoesäure-(4-(5-decylpyrimidin-2-yl)-phenyl-ester.

Beispiel 2

17,0 g 4-(5-Decylpyrimidin-2-yl)-benzoesäure (hergestellt aus 4-(5-Decylpyrimidin-2-yl)-benzonitril durch Verseifung) und 11,1 g 2-Fluor-4-nonylphenol werden in 200 ml Dichlormethan vorgelegt und mit 11,0 g Dicyclohexylcarbodiimid versetzt. Man rührt 4 Stdn. bei 20°, saugt von ausgefallenem Harnstoff ab und engt auf ein kleines Volumen ein. Nach adsorptiver Filtration des Rückstandes über Kieselgel mit Ethylacetat als Elutionsmittel wird 4-(5-Decylpyrimidin-2-yl)-benzoesäure-(2-fluor-4-nonylphenyl)-ester erhalten.

Analog werden erhalten:
4-(5-Butylpyrimidin-2-yl)-benzoesäure-(2-fluor-4-propyl-phenyl)-ester
4-(5-Butylpyrimidin-2-yl)-benzoesäure-(2-fluor-4-pentyl-phenyl)-ester
4-(5-Butylpyrimidin-2-yl)-benzoesäure-(2-fluor-4-hexyl-phenyl)-ester
4-(5-Butylpyrimidin-2-yl)-benzoesäure-(2-fluor-4-heptyl-phenyl)-ester
4-(5-Butylpyrimidin-2-yl)-benzoesäure-(2-fluor-4-octyl-phenyl)-ester
4-(5-Butylpyrimidin-2-yl)-benzoesäure-(2-fluor-4-nonyl-phenyl)-ester
4-(5-Butylpyrimidin-2-yl)-benzoesäure-(2-fluor-4-decyl-phenyl)-ester
4-(5-Pentylpyrimidin-2-yl)-benzoesäure-(2-fluor-4-propyl-phenyl)-ester
4-(5-Pentylpyrimidin-2-yl)-benzoesäure-(2-fluor-4-pentyl-phenyl)-ester
4-(5-Pentylpyrimidin-2-yl)-benzoesäure-(2-fluor-4-hexyl-phenyl)-ester
4-(5-Pentylpyrimidin-2-yl)-benzoesäure-(2-fluor-4-heptyl-phenyl)-ester
4-(5-Pentylpyrimidin-2-yl)-benzoesäure-(2-fluor-4-octyl-phenyl)-ester
4-(5-Pentylpyrimidin-2-yl)-benzoesäure-(2-fluor-4-nonyl-phenyl)-ester
4-(5-Pentylpyrimidin-2-yl)-benzoesäure-(2-fluor-4-decyl-phenyl)-ester
4-(5-Hexylpyrimidin-2-yl)-benzoesäure-(2-fluor-4-propyl-phenyl)-ester
4-(5-Hexylpyrimidin-2-yl)-benzoesäure-(2-fluor-4-pentyl-phenyl)-ester
4-(5-Hexylpyrimidin-2-yl)-benzoesäure-(2-fluor-4-hexyl-phenyl)-ester
4-(5-Hexylpyrimidin-2-yl)-benzoesäure-(2-fluor-4-heptyl-phenyl)-ester
4-(5-Hexylpyrimidin-2-yl)-benzoesäure-(2-fluor-4-octyl-phenyl)-ester
4-(5-Hexylpyrimidin-2-yl)-benzoesäure-(2-fluor-4-nonyl-phenyl)-ester
4-(5-Hexylpyrimidin-2-yl)-benzoesäure-(2-fluor-4-decyl-phenyl)-ester
4-(5-Heptylpyrimidin-2-yl)-benzoesäure-(2-fluor-4-propyl-phenyl)-ester
4-(5-Heptylpyrimidin-2-yl)-benzoesäure-(2-fluor-4-pentyl-phenyl)-ester
4-(5-Heptylpyrimidin-2-yl)-benzoesäure-(2-fluor-4-hexyl-phenyl)-ester
4-(5-Heptylpyrimidin-2-yl)-benzoesäure-(2-fluor-4-heptyl-phenyl)-ester
4-(5-Heptylpyrimidin-2-yl)-benzoesäure-(2-fluor-4-octyl-phenyl)-ester
4-(5-Heptylpyrimidin-2-yl)-benzoesäure-(2-fluor-4-nonyl-phenyl)-ester
4-(5-Heptylpyrimidin-2-yl)-benzoesäure-(2-fluor-4-decyl-phenyl)-ester
4-(5-Octylpyrimidin-2-yl)-benzoesäure-(2-fluor-4-propyl-phenyl)-ester
4-(5-Octylpyrimidin-2-yl)-benzoesäure-(2-fluor-4-pentyl-phenyl)-ester
4-(5-Octylpyrimidin-2-yl)-benzoesäure-(2-fluor-4-hexyl-phenyl)-ester
4-(5-Octylpyrimidin-2-yl)-benzoesäure-(2-fluor-4-heptyl-phenyl)-ester
4-(5-Octylpyrimidin-2-yl)-benzoesäure-(2-fluor-4-octyl-phenyl)-ester
4-(5-Octylpyrimidin-2-yl)-benzoesäure-(2-fluor-4-nonyl-phenyl)-ester
4-(5-Octylpyrimidin-2-yl)-benzoesäure-(2-fluor-4-decyl-phenyl)-ester
4-(5-Nonylpyrimidin-2-yl)-benzoesäure-(2-fluor-4-propyl-phenyl)-ester
4-(5-Nonylpyrimidin-2-yl)-benzoesäure-(2-fluor-4-pentyl-phenyl)-ester
4-(5-Nonylpyrimidin-2-yl)-benzoesäure-(2-fluor-4-hexyl-phenyl)-ester
4-(5-Nonylpyrimidin-2-yl)-benzoesäure-(2-fluor-4-heptyl-phenyl)-ester

4-(5-Nonylpyrimidin-2-yl)-benzoesäure-(2-fluor-4-octyl-phenyl)-ester
4-(5-Nonylpyrimidin-2-yl)-benzoesäure-(2-fluor-4-nonyl-phenyl)-ester
4-(5-Nonylpyrimidin-2-yl)-benzoesäure-(2-fluor-4-decyl-phenyl)-ester
4-(5-Decylpyrimidin-2-yl)-benzoesäure-(2-fluor-4-propyl-phenyl)-ester
4-(5-Decylpyrimidin-2-yl)-benzoesäure-(2-fluor-4-pentyl-phenyl)-ester
4-(5-Decylpyrimidin-2-yl)-benzoesäure-(2-fluor-4-hexyl-phenyl)-ester
4-(5-Decylpyrimidin-2-yl)-benzoesäure-(2-fluor-4-heptyl-phenyl)-ester
4-(5-Decylpyrimidin-2-yl)-benzoesäure-(2-fluor-4-octyl-phenyl)-ester
4-(5-Decylpyrimidin-2-yl)-benzoesäure-(2-fluor-4-nonyl-phenyl)-ester
4-(5-Decylpyrimidin-2-yl)-benzoesäure-(2-fluor-4-decyl-phenyl)-ester.

Beispiel 3

29,8 g 4-(5-Heptylpyrimidin-2-yl)-benzoesäure (erhalten aus 4-(5-Heptylpyrimidin-2-yl)-benzonitril durch Verseifung) und 25,9 g 2-Cyano-4-decylphenol werden unter Zusatz von 21,0 g Dicyclohexylcarbodiimid wie in Beispiel 22 beschreiben miteinander verestert. Man erhält 4-(5-Heptylpyrimidin-2-yl)-benzoesäure-(2-cyan-4-decylphenyl)-ester.

Analog werden erhalten:
4-(5-Propylpyrimidin-2-yl)-benzoesäure-(2-cyan-4-propyl-phenyl)-ester
4-(5-Propylpyrimidin-2-yl)-benzoesäure-(2-cyan-4-pentyl-phenyl)-ester
4-(5-Propylpyrimidin-2-yl)-benzoesäure-(2-cyan-4-hexyl-phenyl)-ester
4-(5-Propylpyrimidin-2-yl)-benzoesäure-(2-cyan-4-heptyl-phenyl)-ester
4-(5-Propylpyrimidin-2-yl)-benzoesäure-(2-cyan-4-octyl-phenyl)-ester
4-(5-Propylpyrimidin-2-yl)-benzoesäure-(2-cyan-4-nonyl-phenyl)-ester
4-(5-Propylpyrimidin-2-yl)-benzoesäure-(2-cyan-4-decyl-phenyl)-ester
4-(5-Pentylpyrimidin-2-yl)-benzoesäure-(2-cyan-4-propyl-phenyl)-ester
4-(5-Pentylpyrimidin-2-yl)-benzoesäure-(2-cyan-4-pentyl-phenyl)-ester
4-(5-Pentylpyrimidin-2-yl)-benzoesäure-(2-cyan-4-hexyl-phenyl)-ester
4-(5-Pentylpyrimidin-2-yl)-benzoesäure-(2-cyan-4-heptyl-phenyl)-ester
4-(5-Pentylpyrimidin-2-yl)-benzoesäure-(2-cyan-4-octyl-phenyl)-ester
4-(5-Pentylpyrimidin-2-yl)-benzoesäure-(2-cyan-4-nonyl-phenyl)-ester
4-(5-Pentylpyrimidin-2-yl)-benzoesäure-(2-cyan-4-decyl-phenyl)-ester
4-(5-Hexylpyrimidin-2-yl)-benzoesäure-(2-cyano-4-propyl-phenyl)-ester
4-(5-Hexylpyrimidin-2-yl)-benzoesäure-(2-cyano-4-pentyl-phenyl)-ester
4-(5-Hexylpyrimidin-2-yl)-benzoesäure-(2-cyano-4-hexyl-phenyl)-ester
4-(5-Hexylpyrimidin-2-yl)-benzoesäure-(2-cyano-4-heptyl-phenyl)-ester
4-(5-Hexylpyrimidin-2-yl)-benzoesäure-(2-cyano-4-octyl-phenyl)-ester
4-(5-Hexylpyrimidin-2-yl)-benzoesäure-(2-cyano-4-nonyl-phenyl)-ester
4-(5-Hexylpyrimidin-2-yl)-benzoesäure-(2-cyano-4-decyl-phenyl)-ester
4-(5-Heptylpyrimidin-2-yl)-benzoesäure-(2-cyan-4-propyl-phenyl)-ester
4-(5-Heptylpyrimidin-2-yl)-benzoesäure-(2-cyan-4-pentyl-phenyl)-ester
4-(5-Heptylpyrimidin-2-yl)-benzoesäure-(2-cyan-4-hexyl-phenyl)-ester
4-(5-Heptylpyrimidin-2-yl)-benzoesäure-(2-cyan-4-heptyl-phenyl)-ester
4-(5-Heptylpyrimidin-2-yl)-benzoesäure-(2-cyan-4-octyl-phenyl)-ester
4-(5-Heptylpyrimidin-2-yl)-benzoesäure-(2-cyan-4-nonyl-phenyl)-ester
4-(5-Heptylpyrimidin-2-yl)-benzoesäure-(2-cyan-4-decyl-phenyl)-ester
4-(5-Octylpyrimidin-2-yl)-benzoesäure-(2-cyan-4-propyl-phenyl)-ester
4-(5-Octylpyrimidin-2-yl)-benzoesäure-(2-cyan-4-pentyl-phenyl)-ester
4-(5-Octylpyrimidin-2-yl)-benzoesäure-(2-cyan-4-hexyl-phenyl)-ester
4-(5-Octylpyrimidin-2-yl)-benzoesäure-(2-cyan-4-heptyl-phenyl)-ester
4-(5-Octylpyrimidin-2-yl)-benzoesäure-(2-cyan-4-octyl-phenyl)-ester
4-(5-Octylpyrimidin-2-yl)-benzoesäure-(2-cyan-4-nonyl-phenyl)-ester
4-(5-Octylpyrimidin-2-yl)-benzoesäure-(2-cyan-4-decyl-phenyl)-ester
4-(5-Nonylpyrimidin-2-yl)-benzoesäure-(2-cyan-4-propyl-phenyl)-ester
4-(5-Nonylpyrimidin-2-yl)-benzoesäure-(2-cyan-4-pentyl-phenyl)-ester
4-(5-Nonylpyrimidin-2-yl)-benzoesäure-(2-cyan-4-hexyl-phenyl)-ester
4-(5-Nonylpyrimidin-2-yl)-benzoesäure-(2-cyan-4-heptyl-phenyl)-ester
4-(5-Nonylpyrimidin-2-yl)-benzoesäure-(2-cyan-4-octyl-phenyl)-ester
4-(5-Nonylpyrimidin-2-yl)-benzoesäure-(2-cyan-4-nonyl-phenyl)-ester
4-(5-Nonylpyrimidin-2-yl)-benzoesäure-(2-cyan-4-decyl-phenyl)-ester
4-(5-Decylpyrimidin-2-yl)-benzoesäure-(2-cyan-4-propyl-phenyl)-ester
4-(5-Decylpyrimidin-2-yl)-benzoesäure-(2-cyan-4-pentyl-phenyl)-ester
4-(5-Decylpyrimidin-2-yl)-benzoesäure-(2-cyan-4-hexyl-phenyl)-ester
4-(5-Decylpyrimidin-2-yl)-benzoesäure-(2-cyan-4-heptyl-phenyl)-ester
4-(5-Decylpyrimidin-2-yl)-benzoesäure-(2-cyan-4-octyl-phenyl)-ester

4-(5-Decylpyrimidin-2-yl)-benzoesäure-(2-cyan-4-nonyl-phenyl)-ester
4-(5-Decylpyrimidin-2-yl)-benzoesäure-(2-cyan-4-decyl-phenyl)-ester.

Beispiel 4

23,4 g 4-Octylbenzoesäure werden gemäß Beispiel 1 mit 15 g Thionylchlorid in das Säurechlorid überführt. Man nimmt das Rohprodukt in 350 ml Dichlormethan auf, setzt 20 ml Triethylamin und 30,0 g 2-Fluor-4-(5-octylpyrimidin-2-yl)-phenol (erhältlich aus 3-Fluor-4-hydroxybenzimidin-hydrochlorid und α-Octyl-β-hydroxyacrylsäuremethylester) zu und erhitzt 8 Stdn. zum Rückfluß. Das Reaktionsgemisch wird mit Eiswasser gewashchen, über Natriumsulfat getrocknet und unter vermindertem Druck vom Lösungsmittel befreit. Es hinterbleibt 4-Octylbenzoesäure-(3-fluor-4-(5-octyl-pyrimidin-2-yl)-phenyl)-ester.

Analog werden erhalten:
4-Butylbenzoesäure-(3-fluor-4-(5-propylpyrimidin-2-yl)-phenyl)-ester
4-Butylbenzoesäure-(3-fluor-4-(5-pentylpyrimidin-2-yl)-phenyl)-ester
4-Butylbenzoesäure-(3-fluor-4-(5-hexylpyrimidin-2-yl)-phenyl)-ester
4-Butylbenzoesäure-(3-fluor-4-(5-heptylpyrimidin-2-yl)-phenyl)-ester
4-Butylbenzoesäure-(3-fluor-4-(5-octylpyrimidin-2-yl)-phenyl)-ester
4-Butylbenzoesäure-(3-fluor-4-(5-nonylpyrimidin-2-yl)-phenyl)-ester
4-Butylbenzoesäure-(3-fluor-4-(5-decylpyrimidin-2-yl)-phenyl)-ester
4-Pentylbenzoesäure-(3-fluor-4-(5-propylpyrimidin-2-yl)-phenyl)-ester
4-Pentylbenzoesäure-(3-fluor-4-(5-pentylpyrimidin-2-yl)-phenyl)-ester
4-Pentylbenzoesäure-(3-fluor-4-(5-hexylpyrimidin-2-yl)-phenyl)-ester
4-Pentylbenzoesäure-(3-fluor-4-(5-heptylpyrimidin-2-yl)-phenyl)-ester
4-Pentylbenzoesäure-(3-fluor-4-(5-octylpyrimidin-2-yl)-phenyl)-ester
4-Pentylbenzoesäure-(3-fluor-4-(5-nonylpyrimidin-2-yl)-phenyl)-ester
4-Pentylbenzoesäure-(3-fluor-4-(5-decylpyrimidin-2-yl)-phenyl)-ester
4-Hexylbenzoesäure-(3-fluor-4-(5-propylpyrimidin-2-yl)-phenyl)-ester
4-Hexylbenzoesäure-(3-fluor-4-(5-pentylpyrimidin-2-yl)-phenyl)-ester
4-Hexylbenzoesäure-(3-fluor-4-(5-hexylpyrimidin-2-yl)-phenyl)-ester
4-Hexylbenzoesäure-(3-fluor-4-(5-heptylpyrimidin-2-yl)-phenyl)-ester
4-Hexylbenzoesäure-(3-fluor-4-(5-octylpyrimidin-2-yl)-phenyl)-ester
4-Hexylbenzoesäure-(3-fluor-4-(5-nonylpyrimidin-2-yl)-phenyl)-ester
4-Hexylbenzoesäure-(3-fluor-4-(5-decylpyrimidin-2-yl)-phenyl)-ester
4-Heptylbenzoesäure-(3-fluor-4-(5-propylpyrimidin-2-yl)-phenyl)-ester
4-Heptylbenzoesäure-(3-fluor-4-(5-pentylpyrimidin-2-yl)-phenyl)-ester
4-Heptylbenzoesäure-(3-fluor-4-(5-hexylpyrimidin-2-yl)-phenyl)-ester
4-Heptylbenzoesäure-(3-fluor-4-(5-heptylpyrimidin-2-yl)-phenyl)-ester
4-Heptylbenzoesäure-(3-fluor-4-(5-octylpyrimidin-2-yl)-phenyl)-ester
4-Heptylbenzoesäure-(3-fluor-4-(5-nonylpyrimidin-2-yl)-phenyl)-ester
4-Heptylbenzoesäure-(3-fluor-4-(5-decylpyrimidin-2-yl)-phenyl)-ester
4-Octylbenzoesäure-(3-fluor-4-(5-propylpyrimidin-2-yl)-phenyl)-ester
4-Octylbenzoesäure-(3-fluor-4-(5-pentylpyrimidin-2-yl)-phenyl)-ester
4-Octylbenzoesäure-(3-fluor-4-(5-hexylpyrimidin-2-yl)-phenyl)-ester
4-Octylbenzoesäure-(3-fluor-4-(5-heptylpyrimidin-2-yl)-phenyl)-ester
4-Octylbenzoesäure-(3-fluor-4-(5-octylpyrimidin-2-yl)-phenyl)-ester
4-Octylbenzoesäure-(3-fluor-4-(5-nonyl-pyrimidin-2-yl)-phenyl)-ester
4-Octylbenzoesäure-(3-fluor-4-(5-decylpyrimidin-2-yl)-phenyl)-ester
4-Nonylbenzoesäure-(3-fluor-4-(5-propylpyrimidin-2-yl)-phenyl)-ester
4-Nonylbenzoesäure-(3-fluor-4-(5-pentylpyrimidin-2-yl)-phenyl)-ester
4-Nonylbenzoesäure-(3-fluor-4-(5-hexylpyrimidin-2-yl)-phenyl)-ester
4-Nonylbenzoesäure-(3-fluor-4-(5-heptylpyrimidin-2-yl)-phenyl)-ester
4-Nonylbenzoesäure-(3-fluor-4-(5-octylpyrimidin-2-yl)-phenyl)-ester
4-Nonylbenzoesäure-(3-fluor-4-(5-nonylpyrimidin-2-yl)-phenyl)-ester
4-Nonylbenzoesäure-(3-fluor-4-(5-decylpyrimidin-2-yl)-phenyl)-ester
4-Decylbenzoesäure-(3-fluor-4-(5-propylpyrimidin-2-yl)-phenyl)-ester
4-Decylbenzoesäure-(3-fluor-4-(5-pentylpyrimidin-2-yl)-phenyl)-ester
4-Decylbenzoesäure-(3-fluor-4-(5-hexylpyrimidin-2-yl)-phenyl)-ester
4-Decylbenzoesäure-(3-fluor-4-(5-heptylpyrimidin-2-yl)-phenyl)-ester
4-Decylbenzoesäure-(3-fluor-4-(5-octylpyrimidin-2-yl)-phenyl)-ester
4-Decylbenzoesäure-(3-fluor-4-(5-nonylpyrimidin-2-yl)-phenyl)-ester
4-Decylbenzoesäure-(3-fluor-4-(5-decyl-pyrimidin-2-yl)-phenyl)-ester.

Beispiel 5

17,0 g 4-(5-Decylpyrimidin-2-yl)-benzoesäure (erhalten aus 4-(5-Decylpyrimidin-2-yl)-benzonitril durch Verseifung), 10,4 g 3-Fluor-4-octylphenol und 10,5 g Dicyclohexylcarbodiimid werden in 280 ml Dichlormethan 14 Stdn. bei 20° gerührt. Es wird von ausgefallenem Dicyclohexylharnstoff abfiltriert und das

Lösungsmittel auf ein kleines Volumen abgezogen, worauf 4-(5-Decylpyrimidin-2-yl)-benzoesäure-(3-fluor-4-octylphenyl)-ester kristallisiert.

Analog dazu werden erhalten:
4-(5-Butylpyrimidin-2-yl)-benzoesäure-(3-fluor-4-propyl-phenyl)-ester
4-(5-Butylpyrimidin-2-yl)-benzoesäure-(3-fluor-4-pentyl-phenyl)-ester
4-(5-Butylpyrimidin-2-yl)-benzoesäure-(3-fluor-4-hexyl-phenyl)-ester
4-(5-Butylpyrimidin-2-yl)-benzoesäure-(3-fluor-4-heptyl-phenyl)-ester
4-(5-Butylpyrimidin-2-yl)-benzoesäure-(3-fluor-4-octyl-phenyl)-ester
4-(5-Butylpyrimidin-2-yl)-benzoesäure-(3-fluor-4-nonyl-phenyl)-ester
4-(5-Butylpyrimidin-2-yl)-benzoesäure-(3-fluor-4-decyl-phenyl)-ester
4-(5-Pentylpyrimidin-2-yl)-benzoesäure-(3-fluor-4-propyl-phenyl)-ester
4-(5-Pentylpyrimidin-2-yl)-benzoesäure-(3-fluor-4-pentyl-phenyl)-ester
4-(5-Pentylpyrimidin-2-yl)-benzoesäure-(3-fluor-4-hexyl-phenyl)-ester
4-(5-Pentylpyrimidin-2-yl)-benzoesäure-(3-fluor-4-heptyl-phenyl)-ester
4-(5-Pentylpyrimidin-2-yl)-benzoesäure-(3-fluor-4-octyl-phenyl)-ester
4-(5-Pentylpyrimidin-2-yl)-benzoesäure-(3-fluor-4-nonyl-phenyl)-ester
4-(5-Pentylpyrimidin-2-yl)-benzoesäure-(3-fluor-4-decyl-phenyl)-ester
4-(5-Hexylpyrimidin-2-yl)-benzoesäure-(3-fluor-4-propyl-phenyl)-ester
4-(5-Hexylpyrimidin-2-yl)-benzoesäure-(3-fluor-4-pentyl-phenyl)-ester
4-(5-Hexylpyrimidin-2-yl)-benzoesäure-(3-fluor-4-hexyl-phenyl)-ester
4-(5-Hexylpyrimidin-2-yl)-benzoesäure-(3-fluor-4-heptyl-phenyl)-ester
4-(5-Hexylpyrimidin-2-yl)-benzoesäure-(3-fluor-4-octyl-phenyl)-ester
4-(5-Hexylpyrimidin-2-yl)-benzoesäure-(3-fluor-4-nonyl-phenyl)-ester
4-(5-Hexylpyrimidin-2-yl)-benzoesäure-(3-fluor-4-decyl-phenyl)-ester
4-(5-Heptylpyrimidin-2-yl)-benzoesäure-(3-fluor-4-propyl-phenyl)-ester
4-(5-Heptylpyrimidin-2-yl)-benzoesäure-(3-fluor-4-pentyl-phenyl)-ester
4-(5-Heptylpyrimidin-2-yl)-benzoesäure-(3-fluor-4-hexyl-phenyl)-ester
4-(5-Heptylpyrimidin-2-yl)-benzoesäure-(3-fluor-4-heptyl-phenyl)-ester
4-(5-Heptylpyrimidin-2-yl)-benzoesäure-(3-fluor-4-octyl-phenyl)-ester
4-(5-Heptylpyrimidin-2-yl)-benzoesäure-(3-fluor-4-nonyl-phenyl)-ester
4-(5-Heptylpyrimidin-2-yl)-benzoesäure-(3-fluor-4-decyl-phenyl)-ester
4-(5-Octylpyrimidin-2-yl)-benzoesäure-(3-fluor-4-propyl-phenyl)-ester
4-(5-Octylpyrimidin-2-yl)-benzoesäure-(3-fluor-4-pentyl-phenyl)-ester
4-(5-Octylpyrimidin-2-yl)-benzoesäure-(3-fluor-4-hexyl-phenyl)-ester
4-(5-Octylpyrimidin-2-yl)-benzoesäure-(3-fluor-4-heptyl-phenyl)-ester
4-(5-Octylpyrimidin-2-yl)-benzoesäure-(3-fluor-4-octyl-phenyl)-ester
4-(5-Octylpyrimidin-2-yl)-benzoesäure-(3-fluor-4-nonyl-phenyl)-ester
4-(5-Octylpyrimidin-2-yl)-benzoesäure-(3-fluor-4-decyl-phenyl)-ester
4-(5-Nonylpyrimidin-2-yl)-benzoesäure-(3-fluor-4-propyl-phenyl)-ester
4-(5-Nonylpyrimidin-2-yl)-benzoesäure-(3-fluor-4-pentyl-phenyl)-ester
4-(5-Nonylpyrimidin-2-yl)-benzoesäure-(3-fluor-4-hexyl-phenyl)-ester
4-(5-Nonylpyrimidin-2-yl)-benzoesäure-(3-fluor-4-heptyl-phenyl)-ester
4-(5-Nonylpyrimidin-2-yl)-benzoesäure-(3-fluor-4-octyl-phenyl)-ester
4-(5-Nonylpyrimidin-2-yl)-benzoesäure-(3-fluor-4-nonyl-phenyl)-ester
4-(5-Nonylpyrimidin-2-yl)-benzoesäure-(3-fluor-4-decyl-phenyl)-ester
4-(5-Decylpyrimidin-2-yl)-benzoesäure-(3-fluor-4-propyl-phenyl)-ester
4-(5-Decylpyrimidin-2-yl)-benzoesäure-(3-fluor-4-pentyl-phenyl)-ester
4-(5-Decylpyrimidin-2-yl)-benzoesäure-(3-fluor-4-hexyl-phenyl)-ester
4-(5-Decylpyrimidin-2-yl)-benzoesäure-(3-fluor-4-heptyl-phenyl)-ester
4-(5-Decylpyrimidin-2-yl)-benzoesäure-(3-fluor-4-octyl-phenyl)-ester
4-(5-Decylpyrimidin-2-yl)-benzoesäure-(3-fluor-4-nonyl-phenyl)-ester
4-(5-Decylpyrimidin-2-yl)-benzoesäure-(3-fluor-4-decyl-phenyl)-ester.

## Beispiel 6

2,6 g 2-Fluor-4-n-hexyloxybenzoylchlorid (erhältlich durch Alkyierung von 3-Fluor-4-cyanophenol mit 1-Bromhexan, alkalische Versiefung des Nitrils zur Säure und Überführung derselben in ihr Chlorid durch Erhitzen mit Thionylchlorid), 2,7 g 5-Hydroxy-2-(4-heptylphenyl)-pyrimidin (hergestellt gemäß Beispiel 18) und 0,8 g Pyridin werden in 20 ml Toluol 3 Stunden auf 100° erhitzt. Nach dem Abkühlen wird das Pyridinhydrochlorid abgesaugt, das Filtrat mit Wasser neutral gewaschen und getrocknet. Abdestillieren des Lösungsmittels und Kristallisation aus Isopropanol ergibt 2-Fluor-4-hexyloxybenzoesäure-(2-(4-heptylphenyl)-pyrimidin-5-yl)-ester.

Analog werden erhalten:
2-Fluor-4-propyloxybenzoesäure-(2-(4-propylphenyl)-pyrimidin-5-yl)-ester
2-Fluor-4-propyloxybenzoesäure-(2-(4-pentylphenyl)-pyrimidin-5-yl)-ester

2-Fluor-4-propyloxybenzoesäure-(2-(4-hexylphenyl)-pyrimidin-5-yl)-ester
2-Fluor-4-propyloxybenzoesäure-(2-(4-heptylphenyl)-pyrimidin-5-yl)-ester
2-Fluor-4-propyloxybenzoesäure-(2-(4-octylphenyl)-pyrimidin-5-yl)-ester
2-Fluor-4-propyloxybenzoesäure-(2-(4-nonylphenyl)-pyrimidin-5-yl)-ester
2-Fluor-4-propyloxybenzoesäure-(2-(4-decylphenyl)-pyrimidin-5-yl)-ester
2-Fluor-4-pentyloxybenzoesäure-(2-(4-propylphenyl)-pyrimidin-5-yl)-ester
2-Fluor-4-pentyloxybenzoesäure-(2-(4-pentylphenyl)-pyrimidin-5-yl)-ester
2-Fluor-4-pentyloxybenzoesäure-(2-(4-hexylphenyl)-pyrimidin-5-yl)-ester
2-Fluor-4-pentyloxybenzoesäure-(2-(4-heptylphenyl)-pyrimidin-5-yl)-ester
2-Fluor-4-pentyloxybenzoesäure-(2-(4-octylphenyl)-pyrimidin-5-yl)-ester
2-Fluor-4-pentyloxybenzoesäure-(2-(4-nonylphenyl)-pyrimidin-5-yl)-ester
2-Fluor-4-pentyloxybenzoesäure-(2-(4-decylphenyl)-pyrimidin-5-yl)-ester
2-Fluor-4-hexyloxybenzoesäure-(2-(4-propylphenyl)-pyrimidin-5-yl)-ester
2-Fluor-4-hexyloxybenzoesäure-(2-(4-pentylphenyl)-pyrimidin-5-yl)-ester
2-Fluor-4-hexyloxybenzoesäure-(2-(4-hexylphenyl)-pyrimidin-5-yl)-ester
2-Fluor-4-hexyloxybenzoesäure-(2-(4-heptylphenyl)-pyrimidin-5-yl)-ester
2-Fluor-4-hexyloxybenzoesäure-(2-(4-octylphenyl)-pyrimidin-5-yl)-ester
2-Fluor-4-hexyloxybenzoesäure-(2-(4-nonylphenyl)-pyrimidin-5-yl)-ester
2-Fluor-4-hexyloxybenzoesäure-(2-(4-decylphenyl)-pyrimidin-5-yl)-ester
2-Fluor-4-heptyloxybenzoesäure-(2-(4-propylphenyl)-pyrimidin-5-yl)-ester
2-Fluor-4-heptyloxybenzoesäure-(2-(4-pentylphenyl)-pyrimidin-5-yl)-ester
2-Fluor-4-heptyloxybenzoesäure-(2-(4-hexylphenyl)-pyrimidin-5-yl)-ester
2-Fluor-4-heptyloxybenzoesäure-(2-(4-heptylphenyl)-pyrimidin-5-yl)-ester
2-Fluor-4-heptyloxybenzoesäure-(2-(4-octylphenyl)-pyrimidin-5-yl)-ester
2-Fluor-4-heptyloxybenzoesäure-(2-(4-nonylphenyl)-pyrimidin-5-yl)-ester
2-Fluor-4-heptyloxybenzoesäure-(2-(4-decylphenyl)-pyrimidin-5-yl)-ester
2-Fluor-4-octyloxybenzoesäure-(2-(4-propylphenyl)-pyrimidin-5-yl)-ester
2-Fluor-4-octyloxybenzoesäure-(2-(4-pentylphenyl)-pyrimidin-5-yl)-ester
2-Fluor-4-octyloxybenzoesäure-(2-(4-hexylphenyl)-pyrimidin-5-yl)-ester
2-Fluor-4-octyloxybenzoesäure-(2-(4-heptylphenyl)-pyrimidin-5-yl)-ester
2-Fluor-4-octyloxybenzoesäure-(2-(4-octylphenyl)-pyrimidin-5-yl)-ester
2-Fluor-4-octyloxybenzoesäure-(2-(4-nonylphenyl)-pyrimidin-5-yl)-ester
2-Fluor-4-octyloxybenzoesäure-(2-(4-decylphenyl)-pyrimidin-5-yl)-ester
2-Fluor-4-nonyloxybenzoesäure-(2-(4-propylphenyl)-pyrimidin-5-yl)-ester
2-Fluor-4-nonyloxybenzoesäure-(2-(4-pentylphenyl)-pyrimidin-5-yl)-ester
2-Fluor-4-nonyloxybenzoesäure-(2-(4-hexylphenyl)-pyrimidin-5-yl)-ester
2-Fluor-4-nonyloxybenzoesäure-(2-(4-heptylphenyl)-pyrimidin-5-yl)-ester
2-Fluor-4-nonyloxybenzoesäure-(2-(4-octylphenyl)-pyrimidin-5-yl)-ester
2-Fluor-4-nonyloxybenzoesäure-(2-(4-nonylphenyl)-pyrimidin-5-yl)-ester
2-Fluor-4-nonyloxybenzoesäure-(2-(4-decyl-phenyl)-pyrimidin-5-yl)-ester
2-Fluor-4-decyloxybenzoesäure-(2-(4-propylphenyl)-pyrimidin-5-yl)-ester
2-Fluor-4-decyloxybenzoesäure-(2-(4-pentylphenyl)-pyrimidin-5-yl)-ester
2-Fluor-4-decyloxybenzoesäure-(2-(4-hexylphenyl)-pyrimidin-5-yl)-ester
2-Fluor-4-decyloxybenzoesäure-(2-(4-heptylphenyl)-pyrimidin-5-yl)-ester
2-Fluor-4-decyloxybenzoesäure-(2-(4-octylphenyl)-pyrimidin-5-yl)-ester
2-Fluor-4-decyloxybenzoesäure-(2-(4-nonylphenyl)-pyrimidin-5-yl)-ester
2-Fluor-4-decyloxybenzoesäure-(2-(4-decylphenyl)-pyrimidin-5-yl)-ester

## Beispiel 7

Aus 29,8 g 4-(5-Heptylpyrimidin-2-yl)-benzoesäure mit 15 g Thionylchlorid analog Beispiel 20 hergestelltes Säurechlorid wird in 330 ml Dichlormethan nach Zugabe von 27,2 g 2,3-Dicyan-4-octyloxyphenol (erhalten aus 2,3-Dicyanhydrochinon und einem Äquivalent Octylbromid in DMF in Gegenwart von Kaliumcarbonat) und 20 ml Triethylamin 12 Stunden bei 20° gerührt. Anschließend wird mit Eiswasser gewaschen, über Natriumsulfat getrocknet und unter vermindertem Druck vom Lösungsmittel befreit. Man erhält so 4-(5-Heptylpyrimidin-2-yl)-benzoesäure-(2,3-dicyan-4-octyloxyphenyl)-ester.

Analog werden erhalten:
4-(5-Propylpyrimidin-2-yl)-benzoesäure-(2,3-dicyan-4-propyloxyphenyl)-ester
4-(5-Propylpyrimidin-2-yl)-benzoesäure-(2,3-dicyan-4-hexyloxyphenyl)-ester
4-(5-Propylpyrimidin-2-yl)-benzoesäure-(2,3-dicyan-4-heptyloxyphenyl)-ester
4-(5-Propylpyrimidin-2-yl)-benzoesäure-(2,3-dicyan-4-octyloxyphenyl)-ester
4-(5-Propylpyrimidin-2-yl)-benzoesäure-(2,3-dicyan-4-nonyloxyphenyl)-ester
4-(5-Propylpyrimidin-2-yl)-benzoesäure-(2,3-dicyan-4-decyloxyphenyl)-ester
4-(5-Pentylpyrimidin-2-yl)-benzoesäure-(2,3-dicyan-4-propyloxyphenyl)-ester
4-(5-Pentylpyrimidin-2-yl)-benzoesäure-(2,3-dicyan-4-hexyloxyphenyl)-ester
4-(5-Pentylpyrimidin-2-yl)-benzoesäure-(2,3-dicyan-4-heptyloxyphenyl)-ester

4-(5-Pentylpyrimidin-2-yl)-benzoesäure-(2,3-dicyan-4-octyloxyphenyl)-ester
4-(5-Pentylpyrimidin-2-yl)-benzoesäure-(2,3-dicyan-4-nonyloxyphenyl)-ester
4-(5-Pentylpyrimidin-2-yl)-benzoesäure-(2,3-dicyan-4-decyloxyphenyl)-ester
4-(5-Hexylpyrimidin-2-yl)-benzoesäure-(2,3-dicyan-4-hexyloxyphenyl)-ester
4-(5-Hexylpyrimidin-2-yl)-benzoesäure-(2,3-dicyan-4-heptyloxyphenyl)-ester
4-(5-Hexylpyrimidin-2-yl)-benzoesäure-(2,3-dicyan-4-octyloxyphenyl)-ester
4-(5-Hexylpyrimidin-2-yl)-benzoesäure-(2,3-dicyan-4-nonyloxyphenyl)-ester
4-(5-Hexylpyrimidin-2-yl)-benzoesäure-(2,3-dicyan-4-decyloxyphenyl)-ester
4-(5-Heptylpyrimidin-2-yl)-benzoesäure-(2,3-dicyan-4-propyloxyphenyl)-ester
4-(5-Heptylpyrimidin-2-yl)-benzoesäure-(2,3-dicyan-4-pentyloxyphenyl)-ester
4-(5-Heptylpyrimidin-2-yl)-benzoesäure-(2,3-dicyan-4-hexyloxyphenyl)-ester
4-(5-Heptylpyrimidin-2-yl)-benzoesäure-(2,3-dicyan-4-octyloxyphenyl)-ester
4-(5-Heptylpyrimidin-2-yl)-benzoesäure-(2,3-dicyan-4-nonyloxyphenyl)-ester
4-(5-Heptylpyrimidin-2-yl)-benzoesäure-(2,3-dicyan-4-decyloxyphenyl)-ester
4-(5-Octylpyrimidin-2-yl)-benzoesäure-(2,3-dicyan-4-propyloxyphenyl)-ester
4-(5-Octylpyrimidin-2-yl)-benzoesäure-(2,3-dicyan-4-pentyloxyphenyl)-ester
4-(5-Octylpyrimidin-2-yl)-benzoesäure-(2,3-dicyan-4-hexyloxyphenyl)-ester
4-(5-Octylpyrimidin-2-yl)-benzoesäure-(2,3-dicyan-4-heptyloxyphenyl)-ester
4-(5-Octylpyrimidin-2-yl)-benzoesäure-(2,3-dicyan-4-octyloxyphenyl)-ester
4-(5-Octylpyrimidin-2-yl)-benzoesäure-(2,3-dicyan-4-nonyloxyphenyl)-ester
4-(5-Octylpyrimidin-2-yl)-benzoesäure-(2,3-dicyan-4-decyloxyphenyl)-ester
4-(5-Nonylpyrimidin-2-yl)-benzoesäure-(2,3-dicyan-4-propyloxyphenyl)-ester
4-(5-Nonylpyrimidin-2-yl)-benzoesäure-(2,3-dicyan-4-pentyloxyphenyl)-ester
4-(5-Nonylpyrimidin-2-yl)-benzoesäure-(2,3-dicyan-4-hexyloxyphenyl)-ester
4-(5-Nonylpyrimidin-2-yl)-benzoesäure-(2,3-dicyan-4-heptyloxyphenyl)-ester
4-(5-Nonylpyrimidin-2-yl)-benzoesäure-(2,3-dicyan-4-octyloxyphenyl)-ester
4-(5-Nonylpyrimidin-2-yl)-benzoesäure-(2,3-dicyan-4-nonyloxyphenyl)-ester
4-(5-Nonylpyrimidin-2-yl)-benzoesäure-(2,3-dicyan-4-decyloxyphenyl)-ester
4-(5-Decylpyrimidin-2-yl)-benzoesäure-(2,3-dicyan-4-propyloxyphenyl)-ester
4-(5-Decylpyrimidin-2-yl)-benzoesäure-(2,3-dicyan-4-pentyloxyphenyl)-ester
4-(5-Decylpyrimidin-2-yl)-benzoesäure-(2,3-dicyan-4-hexyloxyphenyl)-ester
4-(5-Decylpyrimidin-2-yl)-benzoesäure-(2,3-dicyan-4-heptyloxyphenyl)-ester
4-(5-Decylpyrimidin-2-yl)-benzoesäure-(2,3-dicyan-4-octyloxyphenyl)-ester
4-(5-Decylpyrimidin-2-yl)-benzoesäure-(2,3-dicyan-4-nonyloxyphenyl)-ester
4-(5-Decylpyrimidin-2-yl)-benzoesäure-(2,3-dicyan-4-decyloxyphenyl)-ester.

## Beispiel 8

7,9 g 2-Fluor-4-(5-n-heptylpyrimidin-2-yl)-benzoesäure (erhältlich durch alkalische Verseifung von 2-(3-Fluor-4-cyanophenyl)-5-n-heptylpyrimidin) werden in 80 ml Dichlormethan suspendiert. Man gibt 4,3 g trans-4-n-Pentylcyclohexanol und 0,3 g 4-(Dimethylamino)pyridin zu und versetzt bei 5—10° unter Rühren mit 5,8 g Dicyclohexylcarbodiimid. Es wird noch 30 min unter Kühlung, dann über Nacht bei Raumtemperatur gerührt, vom ausgefallenen Harnstoff abfiltriert und das Filtrat zum Rückstand eingeengt. Umkristallisation aus Ethanol ergibt 2-Fluor-4-(5n-heptylpyrimidin-2-yl)-benzoesäure-(trans-4-n-pentylcyclohexyl)ester.

Analog dazu werden erhalten:
2-Fluor-4-(5-butylpyrimidin-2-yl)-benzoesäure-(trans-4-propylcyclohexyl)-ester
2-Fluor-4-(5-butylpyrimidin-2-yl)-benzoesäure-(trans-4-pentylcyclohexyl)-ester
2-Fluor-4-(5-butylpyrimidin-2-yl)-benzoesäure-(trans-4-hexylcyclohexyl)-ester
2-Fluor-4-(5-butylpyrimidin-2-yl)-benzoesäure-(trans-4-heptylcyclohexyl)-ester
2-Fluor-4-(5-butylpyrimidin-2-yl)-benzoesäure-(trans-4-octylcyclohexyl)-ester
2-Fluor-4-(5-butylpyrimidin-2-yl)-benzoesäure-(trans-4-nonylcyclohexyl)-ester
2-Fluor-4-(5-butylpyrimidin-2-yl)-benzoesäure-(trans-4-decylcyclohexyl)-ester
2-Fluor-4-(5-pentylpyrimidin-2-yl)-benzoesäure-(trans-4-propylcyclohexyl)-ester
2-Fluor-4-(5-pentylpyrimidin-2-yl)-benzoesäure-(trans-4-pentylcyclohexyl)-ester
2-Fluor-4-(5-pentylpyrimidin-2-yl)-benzoesäure-(trans-4-hexylcyclohexyl)-ester
2-Fluor-4-(5-pentylpyrimidin-2-yl)-benzoesäure-(trans-4-heptylcyclohexyl)-ester
2-Fluor-4-(5-pentylpyrimidin-2-yl)-benzoesäure-(trans-4-octylcyclohexyl)-ester
2-Fluor-4-(5-pentylpyrimidin-2-yl)-benzoesäure-(trans-4-nonylcyclohexyl)-ester
2-Fluor-4-(5-pentylpyrimidin-2-yl)-benzoesäure-(trans-4-decylcyclohexyl)-ester
2-Fluor-4-(5-hexylpyrimidin-2-yl)-benzoesäure-(trans-4-propylcyclohexyl)-ester, K 77° N 117° I
2-Fluor-4-(5-hexylpyrimidin-2-yl)-benzoesäure-(trans-4-pentylcyclohexyl)-ester
2-Fluor-4-(5-hexylpyrimidin-2-yl)-benzoesäure-(trans-4-hexylcyclohexyl)-ester
2-Fluor-4-(5-hexylpyrimidin-2-yl)-benzoesäure-(trans-4-heptylcyclohexyl)-ester
2-Fluor-4-(5-hexylpyrimidin-2-yl)-benzoesäure-(trans-4-octylcyclohexyl)-ester

2-Fluor-4-(5-hexylpyrimidin-2-yl)-benzoesäure-(trans-4-nonylcyclohexyl)-ester
2-Fluor-4-(5-hexylpyrimidin-2-yl)-benzoesäure-(trans-4-decylcyclohexyl)-ester
2-Fluor-4-(5-heptylpyrimidin-2-yl)-benzoesäure-(trans-4-propylcyclohexyl)-ester, K 61° $S_A$ (49°) N 120° I
2-Fluor-4-(5-heptylpyrimidin-2-yl)-benzoesäure-(trans-4-pentylcyclohexyl)-ester
2-Fluor-4-(5-heptylpyrimidin-2-yl)-benzoesäure-(trans-4-hexylcyclohexyl)-ester
2-Fluor-4-(5-heptylpyrimidin-2-yl)-benzoesäure-(trans-4-heptylcyclohexyl)-ester
2-Fluor-4-(5-heptylpyrimidin-2-yl)-benzoesäure-(trans-4-octylcyclohexyl)-ester
2-Fluor-4-(5-heptylpyrimidin-2-yl)-benzoesäure-(trans-4-nonylcyclohexyl)-ester
2-Fluor-4-(5-heptylpyrimidin-2-yl)-benzoesäure-(trans-4-decylcyclohexyl)-ester
2-Fluor-4-(5-octylpyrimidin-2-yl)-benzoesäure-(trans-4-propylcyclohexyl)-ester
2-Fluor-4-(5-octylpyrimidin-2-yl)-benzoesäure-(trans-4-pentylcyclohexyl)-ester
2-Fluor-4-(5-octylpyrimidin-2-yl)-benzoesäure-(trans-4-hexylcyclohexyl)-ester
2-Fluor-4-(5-octylpyrimidin-2-yl)-benzoesäure-(trans-4-octylcyclohexyl)-ester
2-Fluor-4-(5-octylpyrimidin-2-yl)-benzoesäure-(trans-4-nonylcyclohexyl)-ester
2-Fluor-4-(5-octylpyrimidin-2-yl)-benzoesäure-(trans-4-decylcyclohexyl)-ester
2-Fluor-4-(5-nonylpyrimidin-2-yl)-benzoesäure-(trans-4-propylcyclohexyl)-ester
2-Fluor-4-(5-nonylpyrimidin-2-yl)-benzoesäure-(trans-4-pentylcyclohexyl)-ester
2-Fluor-4-(5-nonylpyrimidin-2-yl)-benzoesäure-(trans-4-hexylcyclohexyl)-ester
2-Fluor-4-(5-nonylpyrimidin-2-yl)-benzoesäure-(trans-4-heptylcyclohexyl)-ester
2-Fluor-4-(5-nonylpyrimidin-2-yl)-benzoesäure-(trans-4-octylcyclohexyl)-ester
2-Fluor-4-(5-nonylpyrimidin-2-yl)-benzoesäure-(trans-4-nonylcyclohexyl)-ester
2-Fluor-4-(5-nonylpyrimidin-2-yl)-benzoesäure-(trans-4-decylcyclohexyl)-ester
2-Fluor-4-(5-decylpyrimidin-2-yl)-benzoesäure-(trans-4-propylcyclohexyl)-ester
2-Fluor-4-(5-decylpyrimidin-2-yl)-benzoesäure-(trans-4-pentylcyclohexyl)-ester
2-Fluor-4-(5-decylpyrimidin-2-yl)-benzoesäure-(trans-4-hexylcyclohexyl)-ester
2-Fluor-4-(5-decylpyrimidin-2-yl)-benzoesäure-(trans-4-octylcyclohexyl)-ester
2-Fluor-4-(5-decylpyrimidin-2-yl)-benzoesäure-(trans-4-nonylcyclohexyl)-ester
2-Fluor-4-(5-decylpyrimidin-2-yl)-benzoesäure-(trans-4-decylcyclohexyl)-ester

## Beispiel 9

Aus 4-(trans-4-Pentylcyclohexyl)-3-fluorbenzoesäure und 4-(5-Nonylpyrimidin-2-yl)-phenol erhält man gemäß Beispiel 1 4-(trans-4-Pentylcyclohexyl)-3-fluorbenzoesäure-4-(5-nonylpyrimidin-2-yl)-phenylester, K 112° N 247° I

Analog werden hergestellt:
4-(trans-4-Propylcyclohexyl)-3-fluorbenzoesäure-4-(5-nonylpyrimidin-2-yl)-phenylester
4-(trans-4-Hexylcyclohexyl)-3-fluorbenzoesäure-4-(5-nonylpyrimidin-2-yl)-phenylester
4-(trans-4-Heptylcyclohexyl)-3-fluorbenzoesäure-4-(5-nonylpyrimidin-2-yl)-phenylester
4-(trans-4-Octylcyclohexyl)-3-fluorbenzoesäure-4-(5-nonylpyrimidin-2-yl)-phenylester
4-(trans-4-Nonylcyclohexyl)-3-fluorbenzoesäure-4-(5-nonylpyrimidin-2-yl)-phenylester
4-(trans-4-Decylcyclohexyl)-3-fluorbenzoesäure-4-(5-nonylpyrimidin-2-yl)-phenylester

## Beispiel 10

Eine flüssigkristalline Phase bestehend aus
31% 2-p-Nonyloxyphenyl-5-nonylpyrimidin
9% 2-p-Decyloxyphenyl-5-octylpyrimidin
14% 2-p-Octyloxyphenyl-5-octylpyrimidin
18% 2-p-Nonyloxyphenyl-5-heptylpyrimidin
13% 2-p-Hepytyloxyphenyl-5-heptylpyrimidin
10% 2-Fluor-4-decyloxybenzoesäure-(4-(5-octylpyrimidin-2-yl)-phenyl)-ester
5% 2-Fluor-4-hexyloxybenzoesäure-(4-(5-decylpyrimidin-2-yl)-phenyl)-ester
hat K/$S_c$ 7°, $S_c$/$S_A$ 57°, $S_A$/N 67°, N/I 70,5°.

## Beispiel 11

Eine flüssigkristalline Phase bestehend aus
28% 2-p-Nonyloxyphenyl-5-nonylpyrimidin
3% 2-p-Nonyloxyphenyl-5-octylpyrimidin
25% 2-p-Heptyloxyphenyl-5-octylpyrimidin
10% 2-p-Heptyloxy-5-heptylpyrimidin
5% 2-p-Decyloxy-5-heptylpyrimidin
9% 2-Fluor-4-decyloxybenzoesäure-(4-(5-octylpyrimidin-2-yl)-phenyl)-ester
10% 3-Fluor-4-heptyloxybenzoesäure-(4-(5-heptylpyrimidin-2-yl)-phenyl)-ester
10% 4-(5-Decylpyrimidin-2-yl)-benzoesäure-(2-fluor-4-nonylphenyl)-ester
hat K/$S_c$ 13°, $S_c$/$S_A$ 64°, $S_A$/N 68°, N/I 83°.

Beispiel 12

Eine flüssigkristalline Phase bestehend aus
30% 2-p-Nonyloxyphenyl-5-nonylpyrimidin
15% 2-p-Decyloxyphenyl-5-octylpyrimidin
15% 2-p-Octyloxyphenyl-5-octylpyrimidin
12% 2-p-Nonyloxyphenyl-5-heptylpyrimidin
10% 4-(5-Octylpyrimidin-2-yl)-benzoesäure-(3-fluor-4-hexylphenyl)-ester
3% 4-(5-Decylpyrimidin-2-yl)-benzoesäure-(2-cyan-4-decylphenyl)-ester
6% 2-Fluor-4-heptyloxybenzoesäure-(2-(4-heptylphenyl)-pyrimidin-5-yl)-ester
9% 3-Fluor-4-hexylbenzoesäure-(2-4-octylphenyl)-pyrimidin-5-yl)-ester
hat K/$S_c$ 16°, $S_c$/$S_A$ 59°, $S_A$/N 63°, N/I 79°.

Beispiel 13

Eine flüssigkristalline Phase bestehend aus
30% 2-p-Nonyloxyphenyl-5-nonylpyrimidin
15% 2-p-Decyloxyphenyl-5-octylpyrimidin
20% 2-p-Octyloxyphenyl-5-octylpyrimidin
15% 2-p-Nonyloxyphenyl-5-heptylpyrimidin
10% 2-p-Heptyloxyphenyl-5-heptylpyrimidin
10% 4-Octylbenzoesäure-(2-fluor-4-(5-octylpyrimidin-2-yl)-phenyl)-ester
hat K/$S_c$ 4°, $S_c$/$S_A$ 22°, $S_A$/N 65°, N/I 74°.

Beispiel 14

Eine flüssigkristalline Phase bestehend aus
33% 4-(5-Hexylpyrimidin-2-yl)-phenyl-(p-hexylbenzyl)-ether
15% 4-(5-Heptylpyrimidin-2-yl)-phenyl-(p-pentylbenzyl)-ether
10% 4-(5-Hexylpyrimidin-2-yl)-phenyl-(p-pentylbenzyl)-ether
3% 4-(5-Nonylpyrimidin-2-yl)-phenyl-(p-hexylbenzyl)-ether
24% 4-(5-Heptylpyrimidin-2-yl)-phenyl-(p-hexylbenzyl)-ether
10% 2-Fluor-4-heptyloxybenzoesäure-(4-(5-octylpyrimidin-2-yl)-phenyl)-ester
5% 4-(5-Decylpyrimidin-2-yl)-benzoesäure-(3-fluor-4-octylphenyl)-ester
hat K/$S_c$ 3°, $S_c$/N 75°, N/I 98°.

Beispiel 15

Eine flüssigkristalline Phase bestehend aus
32% 4-(5-Hexylpyrimidin-2-yl)-phenyl-(p-hexylbenzyl)-ether
16% 4-(5-Heptylpyrimidin-2-yl)-phenyl-(p-pentylbenzyl)-ether
9% 4-(5-Hexylpyrimidin-2-yl)-phenyl-(p-pentylbenzyl)-ether
2% 4-(5-Nonylpyrimidin-2-yl)-phenyl-(p-hexylbenzyl)-ether
18% 4-(5-Nonylpyrimidin-2-yl)-phenyl-(p-pentylbenzyl)-ether
13% 3-Fluor-4-heptyloxybenzoesäure-(4-(5-heptylpyrimidin-2-yl)-phenyl)-ester
5% 4-(5-Decylpyrimidin-2-yl)-benzoesäure-(2-fluor-4-nonylphenyl)-ester
5% 4-(5-Nonylpyrimidin-2-yl)-benzoesäure-(2-cyano-4-octylphenyl)-ester
hat K/$S_c$ −1°, $S_c$/N 77°, N/I 94°.

Beispiel 16

Eine flüssigkristalline Phase bestehend aus
17% 4-(5-Heptylpyrimidin-2-yl)-phenyl-(p-pentylbenzyl)-ether
25% 4-(5-Heptylpyrimidin-2-yl)-phenyl-(p-hexylbenzyl)-ether
13% 4-(Nonylpyrimidin-2-yl)-phenyl-(p-pentylbenzyl)-ether
4% 4-(Nonylpyrimidin-2-yl)-phenyl-(p-hexylbenzyl)-ether
11% 2-Fluor-4-heptylbenzoesäure-(2-(4-nonylphenyl)-pyrimidin-5-yl)-ester
10% 4-Octylbenzoesäure-(2-fluor-4-(5-decylpyrimidin-2-yl)-phenyl)-ester
15% 2-Fluor-4-heptyloxybenzoesäure-(4-(5-heptylpyrimidin-2-yl)-phenyl)-ester
5% 4-(5-Nonylpyrimidin-2-yl)-benzoesäure-(2-fluor-4-nonylphenyl)-ester
hat K/$S_c$ 5°, $S_c$/N 75°, N/I 97°.

Beispiel 17

Eine flüssigkristalline Phase bestehend aus
33% 4-(5-Hexylpyrimidin-2-yl)-phenyl-(p-hexylbenzyl)-ether
18% 4-(5-Heptylpyrimidin-2-yl)-phenyl-(p-pentylbenzyl)-ether
10% 4-(5-Hexylpyrimidin-2-yl)-phenyl-(p-pentylbenzyl)-ether
3% 4-(5-Nonylpyrimidin-2-yl)-phenyl-(p-hexylbenzyl-ether
25% 4-(5-Heptylpyrimidin-2-yl)-phenyl-(p-hexylbenzyl)-ether
4% 3-Fluor-4-octylbenzoesäure-(5-(4-octylphenyl)pyrimidin-2-yl)-ester

7% 4-Heptylbenzoesäure-(2-fluor-4-(5-decylpyrimidin-2-yl)-phenyl)-ester
hat K/$S_C$ 10°, $S_C$/N 77°, N/I 100°.

Beispiel 18

Eine flüssigkristalline Phase bestehend aus
50% 3-Chlor-4-(2-octyloxy)-benzoesäure-(4-(5-nonylpyrimidin-2-yl)-phenylester) und
50% 3-Chlor-4-(2-octyloxy)-benzoesäure-4'-octyloxybiphenyl-4-ylester zeigt
$S_c$* 58° Ch 77° I und $P_s = 91$ nC/cm$^2$ bei 25°.

Beispiel 19

Eine flüssigkristalline Phase bestehend aus
10% 3-Brom-4-octyloxybenzoesäure-4-(5-heptylpyrimidin-2-yl)-phenylester,
10% 3-Brom-4-octylbenzoesäure-4-(5-nonylpyrimidin-2-yl)-phenylester,
20% 3-Brom-4-octyloxybenzoesäure-4-(5-nonylpyrimidin-2-yl)-phenylester,
40% 3-Cyan-4-octyloxybenzoesäure-4-(5-nonylpyrimidin-2-yl)-phenylester, und
20% optisch aktives 3-Chlor-4-(2-octyloxy)-benzoesäure-4-(5-nonylpyrimidin-2-yl)-phenylester
zeigt $S_c$* 78° Ch 105° I.

Beispiel 20

Eine flüssigkristalline Phase bestehend aus
5% 3-Brom-4-octyloxybenzoesäure-4-(5-heptylpyrimidin-2-yl)-phenylester,
5% 3-Brom-4-octylbenzoesäure-4-(5-nonylpyrimidin-2-yl)-phenylester,
20% 3-Brom-4-octyloxybenzoesäure-4-(5-nonylpyrimidin-2-yl)-phenylester,
40% 3-Cyan-4-octyloxybenzoesäure-4-(5-nonylpyrimidin-2-yl)-phenylester,
20% optisch aktives 3-Chlor-4-(2-octyloxy)-benzoesäure-4-(5-nonylpyrimidin-2-yl)-phenylester und
10% 2-Fluor-4-(5-heptylpyrimidin-2-yl)-benzoesäuretrans-4-pyropylcyclohexylester
zeigt $S_c$* 71° $S_A$* 74° Ch 97° I.

Beispiel 21

Eine flüssigkristalline Phase bestehend aus
11% p-(5-Heptylpyrimidin-2-yl)-phenyl-(p-pentylbenzylether),
15% p-(5-Heptylpyrimidin-2-yl)-phenyl-(p-pentylbenzylether),
18% p-(5-Nonylpyrimidin-2-yl)-phenyl-(p-pentylbenzylether),
14% p-(5-Nonylpyrimidin-2-yl)-phenyl-(p-heptylbenzylether),
23% 2-p-Nonyloxyphenyl-5-nonylpyrimidin,
5% p-Hexyloxybenzoesäure-4-(5-heptylpyrimidin-2-yl)-phenylester,
4% p-Octyloxy-m-fluor-benzoesäure-4-(5-octylpyrimidin-2-yl)-phenylester und
10% R-4-(5-Hexylpyrimidin-2-yl)-phenyl-2-chlorpropionat
zeigt K 4° $S_c$* 76° Ch 95° I.

Beispiel 22

Eine flüssigkristalline Phase bestehend aus
3% 2-p-Hexyloxyphenyl-5-heptylpyrimidin,
3% 2-p-Heptyloxyphenyl-5-heptylpyrimidin,
3% 2-p-Octyloxyphenyl-5-heptylpyrimidin,
3% 2-p-Nonyloxyphenyl-5-heptylpyrimidin,
6% 2-p-Hexyloxyphenyl-5-nonylpyrimidin,
15% 2-p-Nonyloxyphenyl-5-nonylpyrimidin,
16% 1-(p-Hexylphenyl)-2-(p-(5-heptylpyrimidin-2-yl)-phenyl)-ethan,
19% 1-(p-Octyl-m-fluorphenyl)-2-(p-(5-octylpyrimidin-2-yl)-phenyl)-ethan,
15% optisch aktives 3-Chlor-4-(2-octyloxy)-benzoesäure-4-(5-nonylpyrimidin-2-yl)-phenylester und
17% optisch aktives 1-p-(2-Octyloxycarbonyl)-phenyl-2-(p-(5-heptylpyrimidin-2-yl)-phenyl)-ethan
zeigt K 9° $S_c$* 74° Ch 89° I.

Beispiel 23

Eine flüssigkristalline Phase bestehend aus
4% 2-p-Heptyloxyphenyl-5-octylpyrimidin,
4% 2-p-Octyloxyphenyl-5-octylpyrimidin,
4% 2-p-Nonyloxyphenyl-5-octylpyrimidin,
16% p-(5-Nonylpyrimidin-2-yl)-phenyl-p-pentylbenzylether,
11% p-(5-Nonylpyrimidin-2-yl)-phenyl-p-heptylbenzylether,
13% p-Octyloxybenzoesäure-5-(p-octylphenyl)-pyrimidin-2-ylester,
16% p-Hexyloxybenzoesäure-5-(p-heptylphenyl)-pyrimidin-2-ylester,
11% p-Heptyloxy-m-fluorbenzoesäure-5-(p-octylphenyl)-pyrimidin-2-ylester und
21% 2-p-Nonyloxyphenyl-5-nonylpyrimidin
zeigt K 5° $S_c$.

Beispiel 24

Eine flüssigkristalline Phase bestehend aus

11% p-(5-Heptylpyrimidin-2-yl)-phenyl-(p-pentylbenzylether),
9% p-(5-Heptylpyrimidin-2-yl)-phenyl-(p-hexylbenzylether),
12% p-(5-Nonylpyrimidin-2-yl)-phenyl-(p-pentylbenzylether),
10% p-(5-Nonylpyrimidin-2-yl)-phenyl-(p-heptylbenzylether),
20% 2-p-Nonyloxyphenyl-5-nonylpyrimidin,
9% 1-(trans-4-Heptylcyclohexyl)-2-(p-(5-octylpyrimidin-2-yl)-phenyl)-ethan,
8% 1-(trans-4-Octylcyclohexyl)-2-(p-(5-nonylpyrimidin-2-yl)-phenyl-ethan,
10% optisch aktives 1-(p-(2-Octyloxy-carbonyl)-phenyl)-2-(p-(5-heptylpyrimidin-2-yl)-phenyl)-ethan
und
11% 1-(p-Heptyloxy-m-fluorphenyl)-2-(p-(5-heptylpyrimidin-2-yl)-phenyl)-ethan

zeigt K 3° $S_c$* 72° Ch 93° I.

## Patentansprüche

1. Stickstoffhaltige heterocyclische Ester der Formel IV

$$R^1—A^1—Z^1—A^2—[Z^2—A^3]_m—R^2 \qquad IV$$

worin

$R^1$ und $R^2$ jeweils eine Alkylgruppe mit 1 bis 12 C-Atomen, worin auch eine oder mehrere nicht benachbarte $CH_2$-Gruppen durch —O—, —CO—, —O—CO—, —O—COO—, —CO—O— und/oder —CH=CH— ersetzt sein können, einer der Reste $R^1$ und $R^2$ auch H, F, Cl, Br oder CN,

$A^1$ ein unsubstituiertes oder durch ein oder zwei F- und/oder Cl- und/oder Br-Atome und/oder $CH_3$- und/oder CN-Gruppen substituiertes Strukturelement ausgewählt aus der Gruppe der Formeln 1 bis 4:

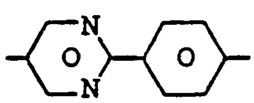, 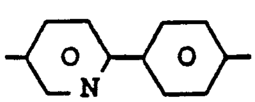, 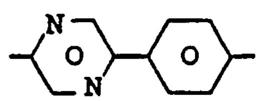, 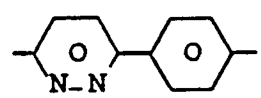

1 2 3 4

$A^2$ und $A^3$ jeweils trans-1,4-Cyclohexylen oder unsubstituierts oder durch ein oder zwei F- und/oder Cl-Atome und/oder Br-Atome und/oder $CH_3$- und/oder CN-Gruppen substituiertes 1,4-Phenylen, worin auch eine oder mehrere CH-Gruppen durch N ersetzt sein können,

$Z^1$ —CO—O— oder —O—CO—,

$Z^2$ —CO—O—, —O—CO—, $—CH_2CH_2—$, $—CH_2O—$, $—O—CH_2—$ oder eine Einfachbindung, und

m 0 oder 1

bedeuten, mit den Maßgaben, daß

(1) die Verbindungen mindestens eine lateral substituierte 1,4-Phenylen-Gruppe enthält und

(2) im Falle

m = 0 und $R^1$-$A^1$-$Z^1$ = Alkyl- 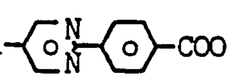 COO

$A^2$ lateral durch Fluor substituiertes 1,4-Phenylen oder in ortho-Stellung zu $R^2$ durch Cl, Br, $CH_3$ oder CN substituiertes 1,4-Phenylen bedeutet.

2. Ester nach Anspruch 1, dadurch gekennzeichnet, daß $R^1$ und $R^2$ Alkyl,—O-Alkyl, Oxaalkyl, —COO—Alkyl, —OCO-Alkyl, —CO-Alkyl oder Alkenyl bedeuten.

3. Ester nach Anspruch 2, dadurch gekennzeichnet, daß $R^1$ und $R^2$ jeweils Methyl, Ethyl, Propyl, Butyl, Pentyl, Hexyl, Heptyl, Octyl, Nonyl, Decyl, Undecyl, Dodecyl, Pentoxy, Hexoxy, Heptoxy, Octoxy, Nonoxy, Decoxy, Undecoxy, Dodecoxy, 2-, 3- oder 4-Oxapentyl, 2-, 3-, 4- oder 5-Oxahexyl. 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl, 1,3-Dioxabutyl (= Methoxymethoxy), 1,3-, 1,4- oder 2,4-Dioxapentyl, 1,3-, 1,4-, 1,5-, 2,4-, 2,5- oder 3,5-Dioxahexyl, 1,3-, 1,4-, 1,5-, 1,6-, 2,4-, 2,5-, 2,6-, 3,5-, 3,6- oder 4,6-Dioxaheptyl, 1,4-Dioxaoctyl, 1,4,7-Trioxaoctyl, 1,4-Dioxanonyl oder 1,4-Dioxadecyl bedeuten.

4. Ester nach mindestens einem Ansprüche 1 bis 3, dadurch gekennzeichnet, daß $Z^1$ —O—CO— bedeutet.

5. Ester nach mindestens einem Ansprüche 1 bis 4, dadurch gekennzeichnet, daß m 0 ist.

6. Ester nach mindestens einem der Ansprüche 1 bis 5, dadurch gekennzeichnet, daß die lateral substituierte 1,4-Phenylen-Gruppe durch ein F-Atom substituiert ist.

7. Ester nach mindestens einem der Ansprüche 1 bis 6, dadurch gekennzeichnet, daß $A^1$ ein Strukturelement der Formel 1 oder 2 ist.

**Revendications**

1. Esters hétérocycliques azotés de formule IV

$$R^1—A^1—Z^1—A^2—[Z^2—A^3]_m—R^2 \qquad IV$$

dans laquelle

$R^1$ et $R^2$ représentent chacun un groupe alkyle en C1—C12 dans lequel un ou plusieurs groupes $CH_2$ non voisins peuvent être remplacés par —O—, —CO—, —O—CO—, —O—COO—, —CO—O— et/ou —CH=CH—, l'un des symboles $R^1$ et $R^2$ pouvant également représenter H, F, Cl, Br ou CN,

$A^1$ représente un élément de structure choisi parmi ceux qui répondent aux formules 1 à 4

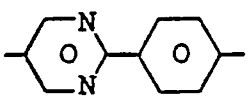 , 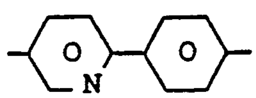 , 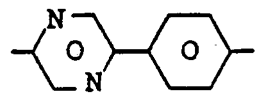 , 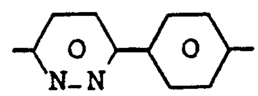

1 2 3 4

non substitué ou substitué par un ou deux atomes de F et/ou de Cl et/ou de Br et/ou groupes $CH_3$ et/ou CN,

$A^2$ et $A^3$ représentent chacun un groupe trans-1,4-cyclohexylène ou un groupe 1,4-phénylène non substitué ou substitué par un ou deux atomes de F et/ou de Cl et/ou atomes de Br et/ou groupes $CF_3$ et/ou CN, et dans lequel un ou plusieurs groupes CH peuvent être remplacés par N,

$Z^1$ représente un groupe —CO—O— ou —O—CO—,

$Z^2$ représente un groupe —CO—O—, —O—CO—, —$CH_2CH_2$—, —$CH_2O$—, —O—$CH_2$— ou une liaison simple et

m est égal à 0 ou 1,

sous réserve que

(1) le composé contient au moins un groupe 1,4-phénylène substitué en position latérale et

(2) dans le cas où

$m = 0$ et $R^1$-$A^1$-$Z^1$ = alkyl- 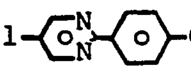 -COO-,

$A^2$ représente un groupe 1,4-phénylène substitué en position latérale par le fluor ou un groupe 1,4-phénylène substitué par Cl, Br, $CH_3$ ou CN en position ortho par rapport à $R^2$.

2. Esters selon la revendication 1, caractérisés en ce que $R^1$ et $R^2$ représentent des groupes alkyle, —O— alkyle, oxa-alkyle, —COO-alkyle, —OCO-alkyle, —CO-alkyle ou alcényle.

3. Esters selon la revendication 2, caractérisés en ce que $R^1$ et $R^2$ représentent chacun un groupe méthyle, éthyle, propyle, butyle, pentyle, hexyle, heptyle, octyle, nonyle, décyle, undécyle, docécyle, pentoxy, hexoxy, heptoxy, octoxy, nonoxy, décoxy, undécoxy, dodécoxy, 2-, 3- ou 4-oxapentyle, 2-, 3-, 4-, ou 5-oxa-hexyle, 2-, 3-, 4-, 5-, ou 6-oxa-heptyle, 2-, 3-, 4-, 5-, 6- ou 7-oxa-octyle, 2-, 3-, 4-, 5-, 6-, 7- ou 8-oxanonyle, 2-, 3-, 4-, 5-, 6-, 7-, 8- ou 9-oxadécyle, 1,3-dioxabutyle (= méthoxyméthoxy), 1,3-, 1,4- ou 2,4-dioxapentyle, 1,3-, 1,4-, 1,5-, 2,4-, 2,5- ou 3,5-dioxa-hexyle, 1,3-, 1,4-, 1,5-, 1,6-, 2,4-, 2,5-, 2,6-, 3,5-, 3,6- ou 4,6-dioxa-heptyle, 1,4-dioxa-octyle, 1,4,7-trioxa-octyle, 1,4-dioxanonyle ou 1,4-dioxadécyle.

4. Esters selon au moins une des revendications 1 à 3, caractérisés en ce que $Z^1$ représente —O—CO—.

5. Esters selon au moins une des revendications 1 à 4, caractérisés en ce que m = 0.

6. Esters selon au moins une des revendications 1 à 5, caractérisés en ce que le groupe 1,4-phénylène substitué en position latérale est substitué par un atome de fluor.

7. Esters selon au moins une des revendications 1 à 6, caractérisés en ce que $A^1$ est un élémente structure de formule I ou 2.

**Claims**

1. Nitrogen-containing heterocyclic esters of the formula IV

$$R^1—A^1—Z^1—A^2—[Z^2—A^3]_m—R^2$$

in which

$R^1$ and $R^2$ are each an alkyl group having 1 to 12 C atoms, in which one or more non-neighbouring $CH_2$

groups may also be replaced by —O—, —CO—, —O—CO—, —O—COO—, —CO—O— and/or —CH=CH—, or one of the radicals $R^1$ and $R^2$ is also H, F, Cl, Br or CN,

A' is a structural element selected from the group comprising the formulae 1 to 4

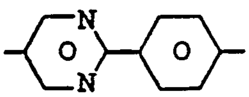 , 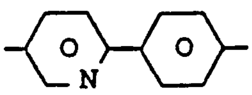 , 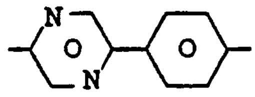 , 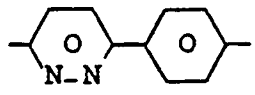

1 2 3 4

which is unsubstituted or substituted by one or two F and/or Cl and/or Br atoms and/or $CH_3$ and/or CN groups,

$A^2$ and $A^3$ are each trans-1,4-cyclohexylene, or 1,4-phenylene, in which one or more CH groups may also be replaced by N, which is unsubstituted or substituted by one or two F and/or Cl atoms and/or Br atoms and/or $CH_3$ and/or CN groups,

$Z^1$ is —CO—O— or —O—CO—,

$Z^2$ is —CO—O—, —O—CO—, —$CH_2CH_2$—, —$CH_2O$—, —O—$CH_2$— or a single bond, and

m is 0 or 1,

with the provisos that

(1) the compound contains at least one laterally substituted 1,4-phenylene group and

(2) in the case of

m = 0 and $R^1$-$A^1$-$Z^1$ = alkyl-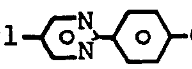-COO-,

$A^2$ is 1,4-phenylene which is laterally substituted by fluorine or is 1,4-phenylene which is substituted, in the ortho position to $R^2$, by Cl, Br, $CH_3$ or CN.

2. Esters according to Claim 1, characterized in that $R^1$ and $R^2$ are alkyl, —O-alkyl, oxaalkyl, —COO-alkyl, —OCO-alkyl, —CO-alkyl or alkenyl.

3. Esters according to Claim 2, characterized in that $R^1$ and $R^2$ are each methyl, ethyl, propyl, butyl, pentyl, hexyl, heptyl, octyl, nonyl, decyl, undecyl, dodecyl, pentoxy, hexoxy, heptoxy, octoxy, nonoxy, decoxy, undecoxy, dodecoxy, 2-, 3- or 4-oxapentyl, 2-, 3-, 4- or 5-oxahexyl, 2-, 3-, 4-, 5- or 6-oxaheptyl, 2-, 3-, 4-, 5-, 6- or 7-oxaoctyl, 2-, 3-, 4-, 5-, 6-, 7- or 8-oxanoyl, 2-, 3-, 4-, 5-, 6-, 7-, 8- or 9-oxadecyl, 1,3-dioxabutyl (= methoxymethoxy), 1,3-, 1,4- or 2,4-dioxapentyl, 1,3-, 1,4-, 1,5-, 2,4-, 2,5- or 3,5-dioxahexyl, 1,3-, 1,4-, 1,5-, 1,6-, 2,4-, 2,5-, 2,6-, 3,5-, 3,6- or 4,6-dioxaheptyl, 1,4-dioxaoctyl, 1,4,7-trioxaoctyl, 1,4-dioxanoyl or 1,4-dioxadecyl.

4. Esters according to at least one of Claims 1 to 3, characterized in that $Z^1$ is —O—CO—.

5. Esters according to at least one of Claims 1 to 4, characterized in that m is O.

6. Esters according to at least one of Claims 1 to 5, characterized in that the laterally substituted 1,4-phenylene group is substituted by an F atom.

7. Esters according to at least one of Claims 1 to 6, characterized in that $A^1$ is a structural element of the formula 1 or 2.